# NEW EUROPEAN PATENT SPECIFICATION

(11) **EP 0 725 137 B2**
(45) Date of publication and mention of the opposition decision: **02.03.2005**
(45) Mention of the grant of the patent: 29.05.2002
(21) Application number: 95928618.8
(22) Date of filing: 18.08.1995
(51) Int. Cl.: C12N 9/02, C12P 7/26

(54) **KETO GROUP INTRODUCING ENZYME, DNA CODING FOR THE SAME, AND PROCESS FOR PRODUCING KETOCAROTENOID**
KETOGRUPPE-EINFÜHRENDES ENZYM, DAFÜR KODIERENDE DNA UND VERFAHREN ZUR HERSTELLUNG VON KETOCAROTENOID
ENZYME D'INTRODUCTION DU GROUPE CETO, ADN CODANT POUR CETTE ENZYME ET PROCEDE D'ELABORATION DE CETOCAROTENOIDE

(30) Priority: 23.08.1994 JP 19877594; 19.09.1994 JP 22379894; 07.03.1995 JP 4726695
(43) Date of publication of application: 07.08.1996
(73) Proprietor: KIRIN BEER KABUSHIKI KAISHA, Tokyo 104-8288 (JP)
(72) Inventor: KAJIWARA, Sususum, Tokyo 158 (JP); MISAWA, Norihiko Kirin Beer Kabushikikaisha, Kanazawa-ku Yokohama-shi, Kanagawa 236 (JP); KONDO, Keiji Kirin Beer Kabushikikaisha, Kanazawa-ku Yokohama-shi, Kanagawa 236 (JP)
(74) Representative: Hansen, Bernd, Dr. Dipl.-Chem.
(86) International application number: PCT/JP1995/001640
(87) International publication number: WO 1996/006172

(56) References cited:
- EP-A- 0 474 347
- WO-A-94/06918
- WO-A-95/18220
- MISAWA N. ET AL.: "Canthaxanthin biosynthesis by the conversion of methylene to keto groups in a hydrocarbon beta-carotene by a single gene" BIOCHEMICAL AND BIOPHYSICAL RESEARCH COMMUNICATIONS, vol. 209, no. 3, 26 April 1995, pages 867-876, XP000602246
- JOHNSON E.A. AND AN G.H.: "Astaxanthin from microbial sources" CRITICAL REVIEWS IN BIOTECHNOLOGY, vol. 11, no. 4, 1991, pages 297-326, XP002069213
- BOUSSIBA S. ET AL.: "Enhancement and determination of Astaxanthin accumulation in green alga Haematococcus pluvialis" METHODS IN ENZYMOLOGY, vol. 213, no. PART A, 1992, pages 386-391, XP000673890
- FEBS LETT., Vol. 364, No. 2, (1995), LOTAN T et al., "Clowing and Expression in Escherichia Coli of the Gene Encoding Beta-C-4-Oxygenase, that Converts Beta-Carotene to the Ketocarotenoid Canthaxanthin in Haematococcus Pluvialis", p. 125-128.
- BIOTECHNOLOGY TECHNIQUES, Vol. 8, No. 1, (1994), MEYER P.S. et al., "Genetic Analysis of Astaxanthin-Overproducing Mutants of Phaffia Rhodozyma Using RAPDs", p. 1-6.

## Description

### FIELD OF THE INVENTION

The present invention relates to a keto group-introducing enzyme necessary for synthesizing ketocarotenoids, such as astaxanthin, which are useful for a red-color enhancing treatment of cultured fishes and shellfishes (such as sea bream, salmon and shrimp) and are also applied to foods as a coloring agent or an antioxidant; a DNA coding for the above enzyme; a recombinant vector comprising the DNA; a microorganism into which the DNA has been introduced; and a method for producing ketocarotenoids using the above microorganism.

### BACKGROUND ART

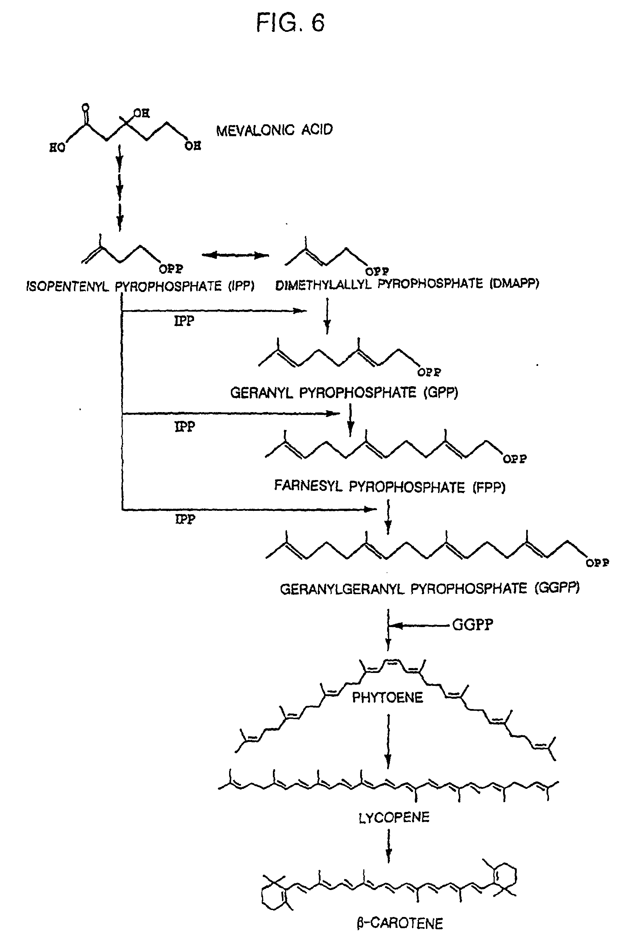

"Ketocarotenoid" is a general term for keto group-containing carotenoid pigments. Carotenoids are synthesized from mevalonic acid as a starting substance via isoprenoid basic biosynthesis pathway which shares an initial part with the synthesis pathway for steroids and other isoprenoids (see Fig. 6). Isopentenyl pyrophosphate (IPP) with 5 carbon atoms, which is a basic unit, generated from the isoprenoid basic biosynthesis pathway condenses with its isomer dimethylallyl pyrophosphate (DMAPP) to produce geranyl pyrophosphate (GPP) with 10 carbon atoms and, in addition, IPP condenses to produce farnesyl pyrophosphate (FPP) with 15 carbon atoms. FPP produces geranylgeranyl pyrophosphate (GGPP) with 20 carbon atoms by condensing with IPP again. Then, GGPPs condense with each other to produce colorless phytoene which is the initial carotenoid. Through a series of unsaturated reactions, phytoene is converted to phytofluene, ζ-carotene, neurosporene and finally to lycopene. Subsequently, lycopene is converted by a cyclization reaction to a β -carotene containing two β -ionone rings. Finally, it is believed that a keto-groups, a hydroxyl group, etc. are introduced into the β -carotene to thereby synthesize astaxanthin, zeaxanthin and the like (see Britton, G., "Biosynthesis of Carotenoids", Plant Pigments, Goodwin, T.W (ed.), London, Academic Press, 1988, pp. 133-182).

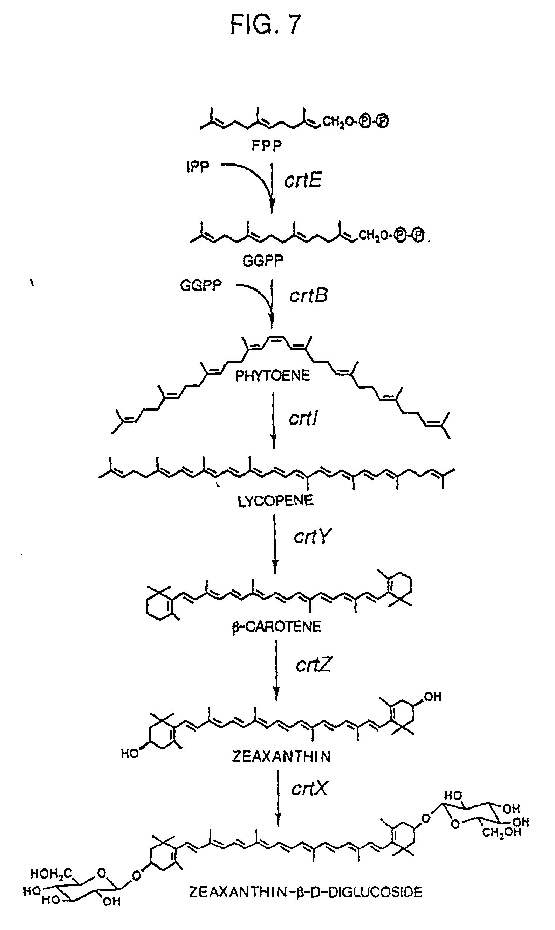

Recently, the present inventors have cloned a group of carotenoid biosynthesis genes of the non-photosynthetic bacterium *Erwinia uredovora* present in plant from the genomic DNA library in *E*. *coli* using its yellow color formation as an indicator. Further, by expressing a various combinations of these genes in microorganisms such as *E. coli*, the inventors has made it possible to produce in microorganisms such as *E. coli* phytoene, lycopene,β -carotene and zeaxanthin which is a yellow carotenoid pigment wherein a hydroxyl group has been introduced into *β*-carotene (see Fig. 7) (Misawa, N., Nakagawa, M., Kobayashi, K., Yamano, S., Izawa, Y., Nakamura; K., and Harashima, K., "Elucidation of the *Erwinia uredovora* Carotenoid Biosynthetic Pathway by Functional Analysis of Gene Products Expressed in *Escherichia coli*", J. Bacteriol., 172, pp. 6704-6712, 1990; Misawa, N., Yamano, S, Ikenaga, H., "Production of β-carotene in *Zymomonas mobilis* and *Agrobacterium tumefaciens* by Introduction of the Biosynthesis Genes from *Erwinia uredovora",* Appl. Environ. Microbiol., 57, pp. 1847-1849, 1991; and Japanese Unexamined Patent Publication No. 3-58786).

On the other hand, astaxanthin which is a red ketokarotenoid is a representative animal carotenoid widely present in marine organisms, e.g. red fishes such as sea bream and salmon, and crustaceans such as crab and shrimp. Since animals generally cannot biosynthesize carotenoids, they have to take in from outside those catotenoids synthesized by microorganisms or plants. For this reason, astaxanthin has been widely used for the purpose of red color enhancing for cultured fishes and shellfishes such as sea bream, salmon and shrimp.

Astaxanthin is also used as a coloring agent for foods. Furthermore, astaxanthin is attracting attention as an antioxidant to remove activated oxygen generated in a body which is causative of a cancer (see Takao Matuno and Wataru Inui, "Physiological Functions and Biological Activities of Carotenoids in Animals", KAGAKU TO SEIBUTU (Chemistry and Organisms), 28, pp. 219-227, 1990).

As sources of astaxanthin supply, there are known crustaceans such as antarctic krill, a culture of the yeast *Phaffia*, a culture of the green alga *Haematococcus* and compounds which are obtained by organic synthesis. However, when crustaceans such as antarctic krill are used, it is difficult to separate astaxanthin from various contaminants, such as lipids, in a recovery and extraction process, which requires a great labor and cost. When a culture of the yeast *Phaffia* is used, the recovery and extraction of astaxanthin also requires a great cost since its cell wall is rigid and yet the production level of astaxanthin is low. In the case of using a culture of the green alga *Haematococcus*, it is necessary to supply to the alga during its cultivation some light which is essential for astaxanthin synthesis. Therefore, appropriate conditions on a location for taking sun light in or cultivation facilities capable of supplying artificial light are required. In addition, it is difficult to separate the produced astaxanthin from mixed up chlorophyl and by-products (fatty acid esters). For these reasons, it has been true that the organism-derived astaxanthin described above cannot compete with those obtained by organic synthesis in cost. However, considering that astaxanthin is used as feed for fishes and shellfishes and as a food additive, an astaxanthin prepared by organic synthesis has some problems with respect to by-products produced in the reaction and yet such an astaxanthin is against the consumers' liking for natural products.

Under circumstances, the development of a method for producing an organism-derived cheap astaxanthin which is safe and can meet the consumers' liking for natural products is desired.

Then, it is believed that the acquisition of a group of genes involved in the biosynthesis of astaxanthin would be very useful, because it is possible to render an optimal microorganism with respect of safety as a food and a potential ability to produce astaxanthin, regardless of whether it has an ability to produce astaxanthin or not, the production ability by introducing into the microorganism the group of astaxanthin synthesis genes and expressing them. In this case, there will occur no problem of the mixing of by-products. In addition, by using techniques of the highly advanced genetic engineering, it will not be difficult to increase the amount of astaxanthin production to a level which exceeds the production amount by organic synthesis. As described above, a group of genes to synthesize up to zeaxanthin have already been obtained by the present inventors from the non-photosynthetic bacterium *Erwinia uredovora*. However, no one has succeeded in obtaining the gene coding for a keto group-introducing enzyme that is necessary for synthesizing astaxanthin, though a number of attempts have been made in many research institute because of the industrial utility of astaxanthin as described above. As to the reasons, it is considered that enzymes located downstream and involved in carotenoid biosynthesis, such as a keto group-introducing enzyme, are membrane proteins and that the purification and measurement of activity of those enzymes have been impossible; therefore, there has been no finding about those enzymes. In particular, as to a keto group-introducing enzyme, not only findings about the enzyme itself but also findings about the gene coding for the enzyme have not been reported at all. Therefore, to date, it has been impossible to produce astaxanthin in a microorganism or the like by using genetic engineering techniques.

### DISCLOSURE OF THE INVENTION

Accordingly, it is an object of the invention to provide the gene coding for a keto group-introducing enzyme which is necessary for producing ketocarotenoids containing keto groups, such as astaxanthin.

It is another object of the invention to provide a keto group-introducing enzyme.

It is still another object of the invention to provide a recombinant vector comprising the gene coding for the keto group-introducing enzyme.

Further, it is still another object of the invention to provide a microorganism into which the gene coding for the keto group-introducing enzyme have been introduced.

Further, it is still another object of the invention to provide a method for producing ketocarotenoids by using the above microorganism into which the gene coding for the keto group-introducing enzyme have been introduced.

The present inventors have made extensive and intensive researces toward solution of the above assignment and, as a result, have succeeded in cloning from the cDNA of the green alga *Haematococcus* the gene coding for a keto group-introducing enzyme, preparing a vector DNA incorporating the gene, introducing the vector DNA into *E. coli*, culturing the resultant *E. coli* in a medium, then collecting the cells from the medium, and extracting ketocarotenoids such as echinenone, canthaxanthin, astaxanthin, 4-ketozeaxanthin and the like. The present invention has been thus achieved. In other words, the invention provides a polypeptide having an enzyme activity to convert the methylene group at position 4 of a β -ionone ring to a keto group. The invention also provides a DNA comprising a base sequence coding for a polypeptide having an enzyme activity to convert the methylene group at position 4 of a β -ionone ring to a keto group. Further, the invention provides a recombinant vector comprising the above DNA. The invention also provides a microorganism into which the above DNA has been introduced. In addition, the invention provides a method for producing ketocarotenoids, comprising culturing in a medium the microorganism into which the DNA has been introduced and extracting ketocarotenoids from the culture cells.

Hereinbelow, the present invention will be described in more detail.

### 1. Keto group-introducing enzyme

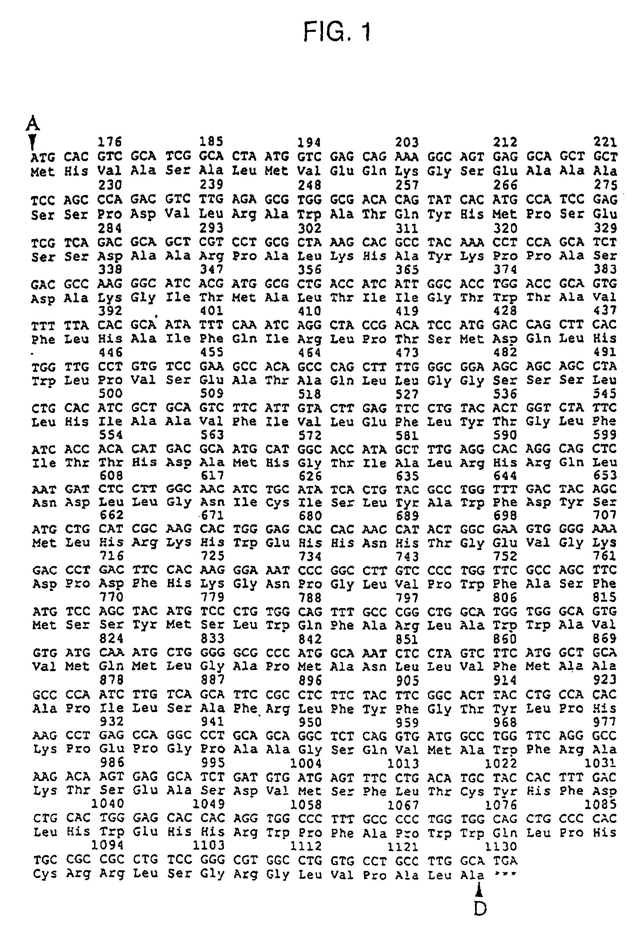

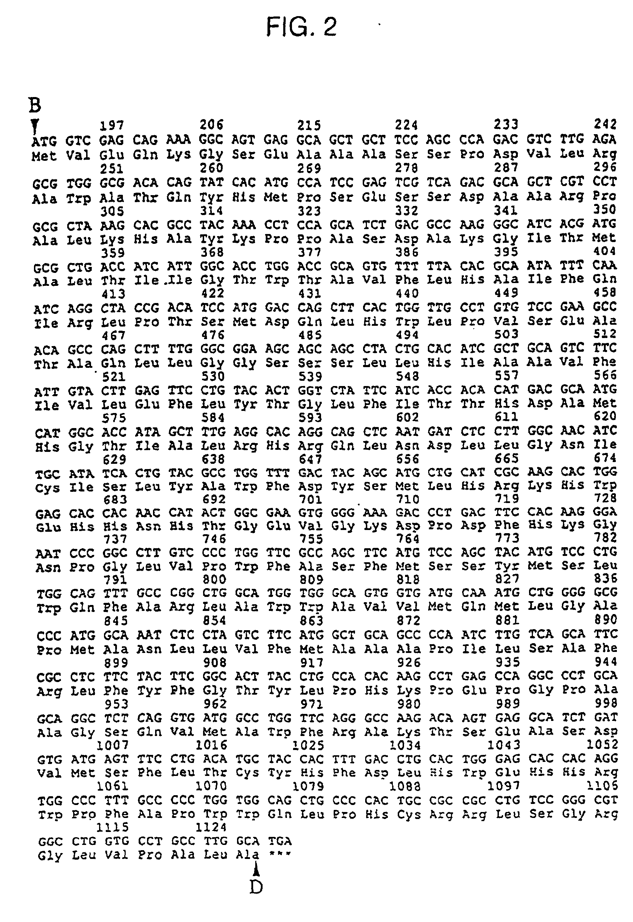

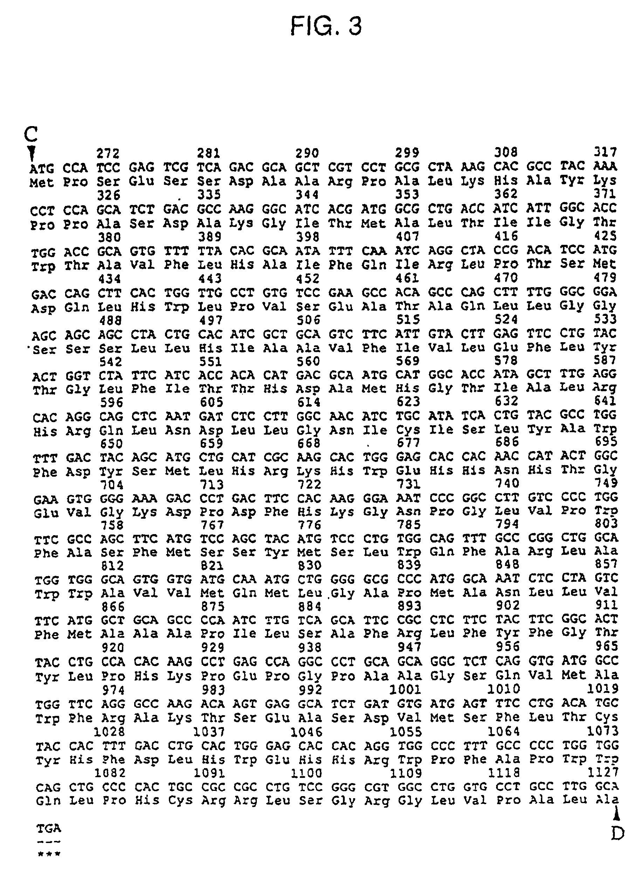

The keto group-introducing enzyme of the invention is a polypeptide having an enzyme activity to convert the methylene group at position 4 of a β-ionone ring to a keto group. This polypeptide may be a polypeptide comprising the amino acid sequence substantially as shown in SEQ ID NO: 1 of the sequence listing (the amino acid sequence from A to D shown in Fig. 1), the amino acid sequence substantially as shown in SEQ ID NO: 2 (the amino acid sequence from B to D shown in Fig. 2) or the amino acid sequence substantially as shown in SEQ ID NO: 3 (the amino acid sequence from C to D shown in Fig. 3). The expression "the amino acid sequence substantially as shown in SEQ ID NO: 1, SEQ ID NO: 2 or SEQ ID NO: 3 of the sequence listing" used here means an amino acid sequence which may have variations such as deletion, substitution, addition, etc. in some of the amino acid residues in the sequence as shown in SEQ ID NO: 1, SEQ ID NO: 2 or SEQ ID NO: 3 of the sequence listing as long as such an amino acid sequence has the enzyme activity to convert the methylene group at position 4 of a β -ionone ring to a keto group, as well as the amino acid sequence as shown in SEQ ID NO: 1, SEQ ID NO: 2 or SEQ ID NO: 3. For example, an amino acid sequence wherein the first amino acid residue (Met) in SEQ ID NO: 1, SEQ ID NO: 2 or SEQ ID NO: 3 of the sequence listing is deleted is included in the above expression.

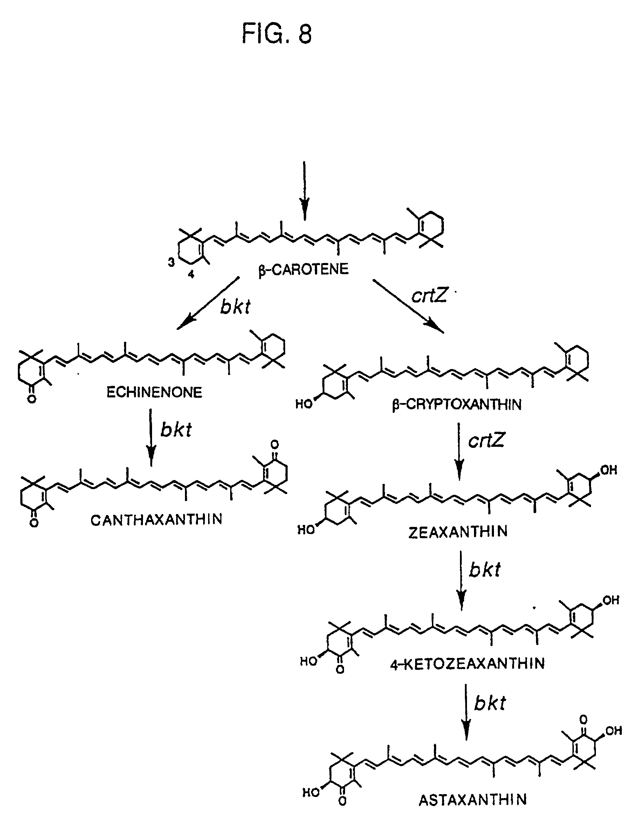

In one embodiment, the keto group-introducing enzyme of the invention is able to synthesize canthaxanthin via echinenone using β -carotene as a substrate. Also, the enzyme of the invention can convert the methylene group at position 4 of 3-hydroxy-β-ionone ring to a keto group. As one specific example of the above, the enzyme of the invention can synthesize astaxanthin via 4-ketozeaxanthin using zeaxanthin as a substrate (see Fig. 8). Since β -carotene and zeaxanthin, which are carotenoids, contain two molecules of β -ionone rings in one molecule, first the methylene group at position 4 of one β -ionone ring is converted to a keto group to produce echinenone and 4-ketozeaxanthin, respectively, and then the methylene group at position 4' (equivalent to position 4) of the other β -ionone ring is converted to a keto group to produce canthaxanthin and astaxanthin, respectively.

### 2. Keto group-introducing enzyme gene (bkt)

The gene coding for the keto group-introducing enzyme of the invention (hereinafter referred to as "bkt") is a DNA comprising a base sequence coding for a polypeptide having an enzyme activity to convert the methylene group at position 4 of a β -ionone ring to a keto group. A typical example of this gene is a bkt gene which can be cloned from the green alga *Haematococcus pluvialis* (NIES-144). This is a DNA comprising a base sequence coding for a polypeptide comprising the amino acid sequence which is substantially shown from A to D in Fig. 1 (the amino acid sequence as shown in SEQ ID NO: 1 of the sequence listing), the amino acid sequence which is substantially shown from B to D in Fig. 2 (the amino acid sequence as shown in SEQ ID NO: 2 of the sequence listing), or the amino acid sequence which is substantially shown from C to D in Fig. 3 (the amino acid sequence as shown in SEQ ID NO: 3 of the sequence listing). Examples for the base sequences coding for the amino acid sequences as shown in SEQ ID NO: 1, SEQ ID NO: 2 and SEQ ID NO: 3 of the sequence listing are given in SEQ ID NOS: 4 and 5, 6 and 7, respectively. The base sequence shown in SEQ ID NO: 4 includes a non-coding region in the upstream of the base sequence shown in SEQ ID NO: 5 which is a coding region. Needless to say, the bkt gene of the invention includes not only the DNAs comprising the base sequences shown in SEQ ID NOS: 4, 5, 6 and 7, but also those DNAs comprising a degenerate isomer coding for the same polypeptide which is different only in degenerate codons.

The bkt gene product (hereinafter referred to as "BKT"), i.e., the keto group-introducing enzyme of the present invention has, as described above, an enzyme activity to convert the methylene group at position 4 of a β -ionone ring in a compound containing β-ionone rings to a keto group. In one embodiment, BKT can synthesize canthaxanthin via echinenone using β -carotene as a substrate (see Fig. 8). Further, BKT can also convert the methylene group at position 4 of 3-hydroxy-β-ionone -ionone ring to a keto group. For example, BKT can synthesize astaxanthin via 4-ketozeaxanthin using zeaxanthin as a substrate (see Fig. 8). A polypeptide having such an enzyme activity and the DNA coding for it have not been known. This polypeptide and the DNA coding therefor do not have an overall homology with any of the polypeptides and DNAs which have been known to date. In addition, not limited to the conversion in a β -ionone ring or 3-hydroxy-β-ionone ring, there has been no finding that one enzyme converts a methylene group immediately to a keto group.

On the other hand, by using the carotenoid synthesis gene group of crtE, crtB, crtl and crtY from the non-photosynthetic bacteria *Erwinia*, it is possible to render a microorganism such as *E. coli* an ability to produce β-carotene. By using crtZ gene in addition to the above four genes, it is possible to render a microorganism such as *E. coli* an ability to produce zeaxanthin (see Fig. 7 and WO91/13078, *supra).*

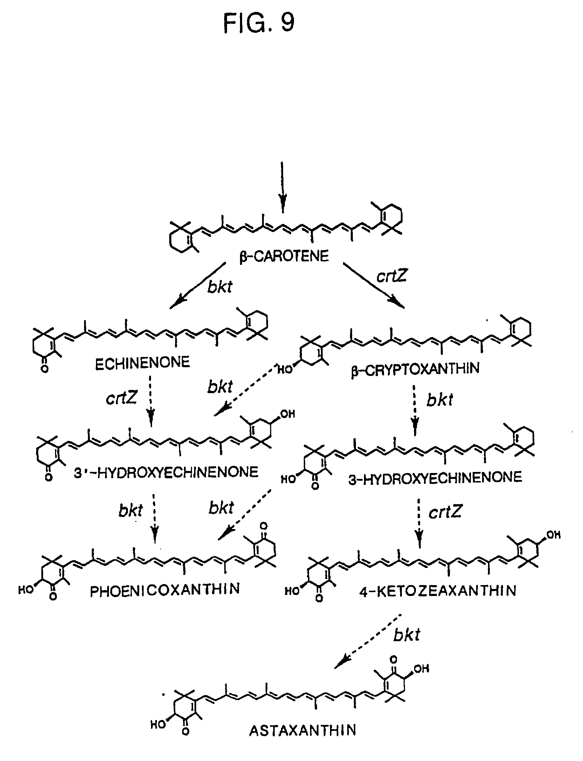

Accordingly, since β -carotene and zeaxanthin (which are substrates for BKT) are supplied by these crt gene group from *Erwinia,* when the DNA of the invention (bkt gene) is further introduced to a microorganism such as *E. coli* carrying the crt gene group from *Erwinia*, it will become possible for a β-carotene producing microorganism to produce canthaxanthin via echinenone and for a zeaxanthin producing microorganism to produce astaxanthin via 4-ketozeaxanthin (see Fig. 8). However, in a zeaxanthin producing microorganism, β-cryptoxanthin is contained in an extremely small amount as an intermediate. Therefore, in addition to the major metabolic pathway described above, there may be another pathway producing astaxanthin from β -cryptoxanthin via 3-hydroxyechinenone and 4-ketozeaxanthin, and still another pathway producing phoenicoxanthin from β -cryptoxanthin via 3-hydroxyechinenone or 3'-hydroxyechinenone. As products of these minor pathways, it is considered that 3'-hydroxyechinenone, 3-hydroxyechinenone and phoenicoxanthin can be produced (see Fig. 9).

### 3. Acquisition of the DNA

One means to obtain the DNA comprising a base sequence coding for an amino acid sequence of the keto group-introducing enzyme (BKT) of the invention is to chemically synthesize at least a portion of the DNA chain according to the conventional nucleic acid synthesizing methods. However, considering the length of sequence, it is preferable not to use the chemical synthesis but to obtain mRNA from the green algae *Haematococcus (Haematococcus pluvialis* and *Haematococcus lacustris* are representative varieties), prepare a cDNA library therefrom using *E. coli*, and obtain the DNA from this library by conventional methods used in the field of genetic engineering, e.g., the hybridization method with appropriate probes or the expression cloning method which the inventors have employed.

Specifically, the total RNA of *Haematococcus pluvialis* is separated and poly A ⁺ RNA is purified using Oligotex-dT30 Super (Takara Shuzo). Using this poly A⁺ RNA as a template, cDNA is synthesized with the reverse transcriptase Superscript™ RT (Gibco BRL) and then double-stranded cDNA is synthesized with *E. coli* DNA ligase, *E. coli* DNA polymerase and *E. coli* DNA RNase H (all manufactured by (Gibco BRL). The synthesized cDNA is incorporated in an *E. coli* expression vector pSPORT1 (Gibco BRL) and a cDNA library is prepared. Using this cDNA library, a β -carotene producing *E. coli* ( *E. coli* carrying the crt gene group of *Erwinia* as described above) is transformed. From the changes in color tone in the resultant transformants, those microorganisms carrying the keto group-introducing enzyme gene are screened. This method utilizes the phenomenon that the color tone of *E. coli* changes from a β -carotene-derived yellow to a canthaxanthin-derived red when a keto group has been introduced and canthaxanthin, one of ketocarotenoids, has been synthesized. From the transformed red *E. coli* thus obtained, a plasmid having a cDNA of interest is isolated and the cDNA is re-linked to *E. coli* vectors pBluescript II® SK+ and pBluescript II® KS+ (Stratagene). With these plasmids, deletion variants having various lengths of deletions are produced and the base sequences of the variants are determined.

### 4. DNAs which hybridize with the bkt gene

To date, several varieties of the green algae *Haematococcus* have been isolated and identified, and all of them are considered to synthesize ketocarotenoids such as astaxanthin. In yeast, *Phaffia rhodozyma* which is also an eucaryote has been reported to synthesize ketocarotenoids such as astaxanthin (Johnson, E.A. and An, G.-Hwan, "Astaxanthin from Microbial Sources", Critical Reviews in Biotechnology, 11, pp. 297-326, 1991). It is possible to obtain other genes of keto group-introducing enzymes from the above astaxanthin producing algae or microorganisms by using as a probe the *Haematococcus pluvialis* NIES-144 bkt gene as described above and carrying out hybridization by utilizing their homology. The present inventors have selected from those *Haematococcus* capable of synthesizing astaxanthin two varieties which are different from *Haematococcus pluvialis* NIES-144 in assimilation property and phenotype against light. They are *Haematococcus lacustris* UTEX 294 (released from the Culture Collection of Algae at the University of Texas at Austin) and *Haematococcus lacustris* C-392 [released from the Microorganisms and Micro-algae Center belonging to the Applied Microorganism Laboratory (the current Molecular Cell Biology Laboratory), the University of Tokyo]. The genomic DNAs from these varieties were prepared and Southern hybridization was conducted using as a probe the *Haematococcus pluvialis* NIES-144 bkt gene. The results were as expected by the inventors. The bkt probe strongly hybridized with specific DNA fragments derived from either of the genomic DNA. Therefore, the present invention includes those DNAs which hybridize with the above-described DNAs (SEQ ID NOS: 4, 5, 6 and 7).

### 5. Transformation of a microorganism such as E. coli

By introducing the DNA of the invention as a foreign gene into an appropriate microorganism such as bacteria (e.g., *E. coli, Zymomonas mobilis, Agrobacterium tumefaciens),* yeast (e.g. *Saccharomyces cerevisiae), etc*. and expressing it, various ketocarotenoids can be produced.

Hereinbelow, the method for introducing a foreign gene into a preferable microorganism will be described briefly. With respect to procedures or methods for introducing a foreign gene into a microorganism such as *E. coli* and expressing the gene, conventional ones used in the field of genetic engineering may be used, as well as the procedures described herein. For example, procedures or methods according to those described in "Vectors for Cloning Genes", Methods in Enzymology, 216, pp. 469-631, 1992, Academic Press and " Other Bacterial Systems", Methods in Enzymology, 204, pp. 305-636, 1991, Academic Press may be used.

### <Introduction of the gene into E. coli>

As a method for introducing a foreign gene into *E. coli*, there are several established, effective methods which may be used, such as Hanahan's method and the rubidium method (see, for example, Chapter 1, pp. 74-84, Sambrook, J., Fritsch, E.F., Maniatis, T., "Molecular Cloning, A Laboratory Manual", Cold Springs Harbor Laboratory Press, 1989). For the expression of a foreign gene in *E. coli*, it is preferable, for example, to introduce into *E. coli* a lac promoter-containing *E. coli* expression vector into which the foreign gene has been inserted according to conventional methods (see, for example, Chapter 17, pp. 3-41, "Molecular Cloning, A Laboratory Manual" *supra* ). The present inventors have inserted the *Haematococcus* bkt gene into the *E. coli* cDNA expression vector pSPORT1 (Gibco BRL) having a lac promoter etc. in a direction so that the inserted gene undergoes a read through of the transcription of the lac promoter, and then introduced the resultant vector into *E. coli.*

### <Introduction of the gene into yeast>

As a method for introducing a foreign gene into the yeast *Sacchromyces cerevisiae*, there are established methods such as the lithium method which may be used (for example, see "KOHBONO NYUHBAIOTEKUNOROJIH (New Biotechnology of Yeast)" edited by the Bioindustry Association under the supervision of Y. Akiyama, published by Igaku Shuppan Center). For the expression of a foreign gene in yeast, it is preferable to construct an expression cassette using a promoter and a terminator such as PGK and GPD, in which cassette the foreign gene is inserted between the promoter and the terminator so that the gene undergoes a read through of the transcription. Then, this expression cassette is inserted into a vector for *S. cerevosiae ,* for example, YRp system vector (a yeast multicopy vector making the ARS sequence in yeast chromosomes as a replication origin), YEp system vector (a yeast multicopy vector having a replication origin of yeast 2 µm DNA), Ylp system vector (a vector to be incorporated in yeast chromosomes, not having a replication origin of yeast), etc. and the resultant vector is introduced into the yeast (see "New Biotechnology of Yeast", *supra* ; Japan Agricultural & Horticultural Chemistry Association ABC Series "BUSSHITU SEISAN NOTAMENO IDENSHIKOUGAKU (Genetic Engineering for the Production of Substances)", Asakura Shoten Co., Ltd.; and Yamano, S., Ishii, T., Nakagawa, M., Ikenaga, H., and Misawa, N., "Metabolic Engineering for Production of β -carotene and Lycopene in *Sacchromyces cerevisiae*", Biosci. Biotech. Biochem., 58, pp. 1112-1114, 1994).

### <Introduction of the gene into Zymomonas mobilis>

The introduction of a foreign gene into the ethanol producing bacterium *Zymomonas mobilis* can be achieved by the conjugative transfer method which is commonly used for Gram-negative bacteria. For the expression of a foreign gene in *Zymomonas mobilis*, it is preferable, for example, to introduce into *Zymomonas mobilis* an expression vector into which the foreign gene has been inserted (e.g. , vector pZA22 for *Zymomonas mobilis*) (see Nakamura, K., "Molecular Breeding of *Zymomonas* Bacteria", Journal of Japan Agricultural & Horticultural Chemistry Association, 63, pp. 1016-1018, 1989; and Misawa, N., Yamano, S, Ikenaga, H., "Production of β-carotene in *Zymomonas mobilis* and *Agrobacterium tumefaciens* by Introduction of the Biosynthesis Genes from *Erwinia uredovora",* Appl. Environ. Microbiol., 57, pp. 1847-1849, 1991).

### <Introduction of the gene into Agrobacterium tumefaciens>

The introduction of a foreign gene into the plant pathogenic bacterium *Agrobacterium tumefaciens* can be achieved by the conjugative transfer method which is commonly used for Gram-negative bacteria. For the expression of a foreign gene in *Agrobacterium tumefaciens,* it is preferable, for example, to introduce into *Agrobacterium tumefaciens* an expression vector into which the foreign gene has been inserted (e.g., vector pBI121 for *Agrobacterium tumefaciens)* (see Misawa, N., Yamano, S, Ikenaga, H., "Production of -carotene in *Zymomonas mobilis* and *Agrobacterium tumefaciens* by Introduction of the Biosynthesis Genes from *Erwinia uredovora*". Appl. Environ. Microbiol., 57, pp. 1847-1849, 1991).

### 6. Production of ketocarotenoids by microorganisms (expression of the bkt gene)

By using the techniques or methods as described above to introduce a foreign gene into a microorganism, it is possible to introduce into a microorganism a *Haematococcus*-derived group of ketocarotenoid (including astaxanthin) synthesis genes and express them.

Farnesyl pyrophosphate (FPP) is not only a substrate of carotenoids but is also a common substrate of other isoprenoids such as sesquiterpene, triterpene, sterol, hopanol, etc. Generally, microorganisms including those which cannot synthesize carotenoids synthesize other isoprenoids. Therefore, basically every microorganism is supposed to have FPP as an intermediary metabolite. On the other hand, using FPP as a substrate, the carotenoid synthesis gene group of the non-photosynthetic *Erwinia* is able to synthesize the substrates of the *Haematococcus* bkt gene product, i.e., up to β -carotene and zeaxanthin (see Fig. 7). The present inventors have introduced the *Erwinia* crt gene group not only into *E. coli* but also the microorganisms described above, (i.e. the yeast *Saccharomyces cerevisiae*, the ethanol producing bacterium *Zymomonas mobilis* and the plant pathogenic bacterium *Agrobacterium tumefaciens*) and confirmed that these microorganism have become able to produce carotenoids such as β -carotene as expected (see Yamano, S., Ishii, T., Nakagawa, M., Ikenaga, H., and Misawa, N., " Metabolic engineering for production of β -carotene and lycopene in *Saccharomyces cerevisiae*", Biosci., Biotech. Biochem., 58, p. 1112-1114, 1994; Misawa, N., Yamano, S, Ikenaga, H., "Production of β-carotene in *Zymomonas mobilis* and *Agrobacterium tumefaciens* by introduction of the biosynthesis genes from *Erwinia uredovora",* Appl. Environ. Microbiol., 57, pp. 1847-1849, 1991; and Japanese Unexamined Patent Publication No. 3-58786).

Accordingly, by introducing a combination of *Erwinia*-derived carotenoid synthesis genes with the DNA of the invention (which is typically the *Haematococcus*-derived carotenoid synthesis gene bkt) into the same microorganism simultaneously, it becomes possible to produce ketocarotenoids such as astaxanthin in all of those microorganisms wherein a gene introduction/expression system has been established. Alternatively, by introducing the DNA of the invention into a microorganism which inherently has carotenoid synthesis genes or a microorganism into which carotenoid synthesis genes have been already introduced, it is also possible to produce ketocarotenoids in the above microorganism. Hereinbelow, the production of various ketocarotenoids by microorganisms will be described.

### <Production of canthaxanthin and echinenone>

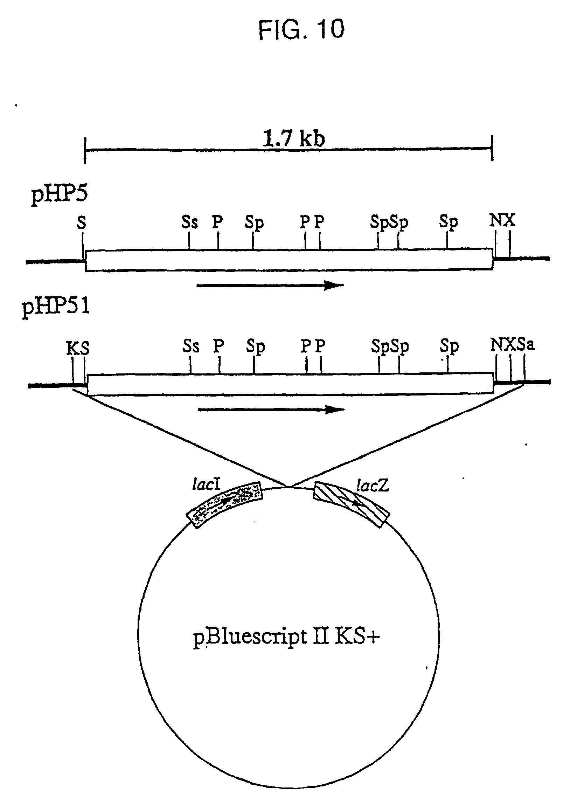

By introducing into a microorganism, such as *E. coli*, the *Erwinia uredovora* crtE, crtB, crtl and crtY genes necessary for the synthesis of β -carotene and the *Haematococcus* bkt gene which is a keto group-introducing enzyme gene and expressing them, it is possible to allow the microorganism to produce canthaxanthin as a final product. Furthermore, by regulating the level of expression of the bkt gene or the like, echinenone which is a synthetic intermediate can also be obtained. For example, in order to produce canthaxanthin and echinenone in *E. coli*, both a first plasmid (e.g., pACCAR16ΔcrtX) obtainable by inserting into an *E. coli* vector (e.g., pACYC184) a fragment containing the *Erwinia uredovora* crtE, crtB, crtl and crtY genes and a second plasmid [e.g., pHP51 (see Fig. 10)] obtainable by inserting into an *E. coli* vector (e.g., pBluescript II KS⁺) a fragment containing the *Haematococcus* bkt gene are introduced into *E. coli* (e.g., JM101). The resultant *E. coli* is cultured in LB medium, 2YT medium or the like containing ampicillin and chloramphenicol under culture conditions at 30-37 °C until the stationary phase. Then, cells are harvested and carotenoid pigments are extracted by using an organic solvent such as acetone. Canthaxanthin and echinenone may be contained in the carotenoid pigments thus obtained.

### <Production of astaxanthin and 4-ketozeaxanthin>

By introducing into a microorganism, such as *E. coli*, the *Erwinia uredovora* crtE, crtB, crtl, crtY and crtZ genes necessary for the synthesis of zeaxanthin and the *Haematococcus* bkt gene which is a keto group-introducing enzyme gene and expressing them, it is possible to allow the microorganism to produce astaxanthin as a final product. Furthermore, by regulating the level of expression of the bkt gene or the like, 4-ketozeaxanthin which is a synthetic intermediate can also be obtained. For example, in order to produce astaxanthin and 4-ketozeaxanthin in *E. coli*, both a first plasmid (e.g., pACCAR25ΔcrtX) obtainable by inserting into an *E. coli* vector (e.g., pACYC184) a fragment containing the *Erwinia uredovora* crtE, crtB, crtl, crtY and crtZ genes and a second plasmid (e.g., pHP51) obtainable by inserting into an *E. coli* vector (e.g., pBluescript II KS⁺) a fragment containing the *Haematococcus* bkt gene are introduced into *E. coli* (e.g., JM101). The resultant *E. coli* is cultured in, for example, LB medium or 2YT medium containing ampicillin and chloramphenicol under culture conditions at 30-37 °C until the stationary phase. Then, cells are harvested and carotenoid pigments are extracted by using an organic solvent such as acetone. Astaxanthin and 4-ketozeaxanthin may be contained in the carotenoid pigments thus obtained.

### <Production of 3'-hydroxyechinenone, 3-hydroxyechinenone and phoenicoxanthin>

By introducing into a microorganism, such as *E. coli*, the *Erwinia uredovora* crtE, crtB, crtl, crtY and crtZ genes necessary for the synthesis of zeaxanthin and the *Haematococcus* bkt gene which is a keto group-introducing enzyme gene and expressing them, it is possible to allow the microorganism to produce astaxanthin and 4-ketozeaxanthin as major products. However, as minor intermediary metabolites, 3'-hydroxyechinenone, 3-hydroxyechinenone and phoenicoxanthin should be present in the pathway.

Methods for producing these pigments are similar to those methods described above. For details, see the Examples.

### 7. Deposit of the microorganism

The *E. coli* DH5_{α} into which plasmid pHP51 incorporating the isolated bkt gene (i.e., the DNA of the invention) has been introduced was deposited at the National Institute of Bioscience and Human-technology, Agency of Industrial Science and Technology, as follows:
Designation for identification assigned by the depositor:
   DH5_{α} (pHP51)
Accession Number: FERM BP-4757
Date of Deposit: July 26, 1994

### BRIEF DESCRIPTION OF DRAWINGS

Fig. 1 shows the base sequence of a keto group-introducing enzyme gene (bkt) from the green alga *Haema tococcus pluvialis* NIES-144 and the amino acid sequence of a polypeptide encoded by the above base sequence.

Fig. 2 shows the base sequence of a keto group-introducing enzyme gene (bkt) from the green alga *Haematococcus pluvialis* NIES-144 and the amino acid sequence of a polypeptide encoded by the the base sequence.

Fig. 3 shows the base sequence of a keto group-introducing enzyme gene (bkt) from the green alga *Haema tococcus pluvialis* NIES-144 and the amino acid sequence of a polypeptide encoded by the the base sequence.

In Figs. 1 to 3 above, the initiation codons are different ones.

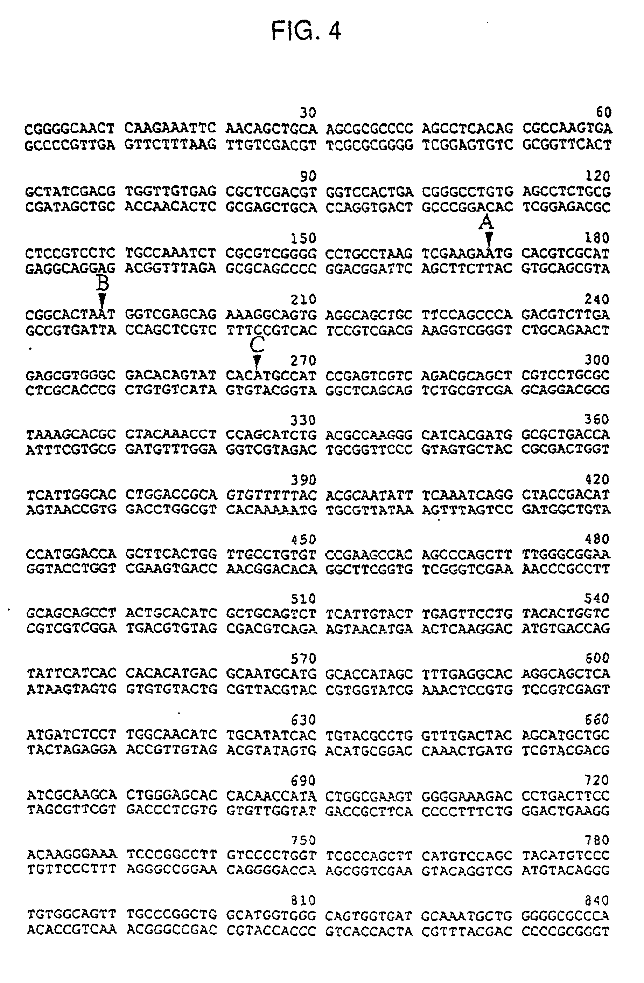

Fig. 4 shows the base sequence of a DNA chain comprising a keto group-introducing enzyme gene (bkt) from the green alga *Haematococcus pluvialis* NIES-144). A, B and C in the Fig. show the positions of the initiation codons.

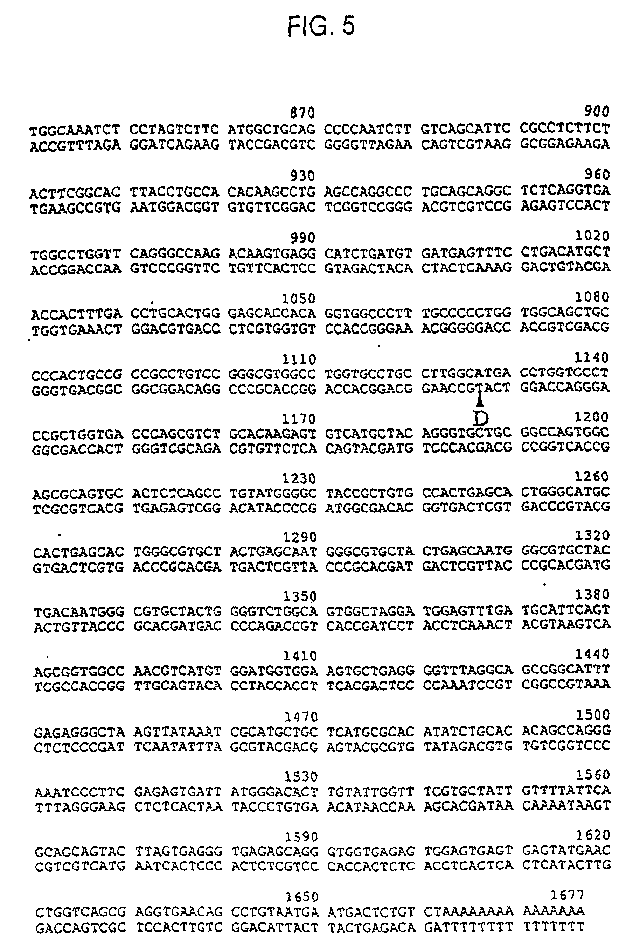

Fig. 5 shows a sequence which follows the one shown in Fig. 4.

Fig. 6 shows a carotenoid biosynthesis pathway up to β-carotene.

Fig. 7 shows the carotenoid biosynthesis pathway of the non-photosynthetic *Erwinia uredovora* as well as the functions of carotenoid synthesis genes.

Fig. 8 shows the functions of the keto group-introducing enzyme gene (bkt) from the green alga *Haematococcus pluvialis* NIES-144, the functions of the hydroxyl group-introducing enzyme gene (crtZ) from the non-photosynthetic *Erwinia uredovora* and major ketocarotenoid biosynthesis pathways.

Fig. 9 shows the functions of the keto group-introducing enzyme gene (bkt) from the green alga *Haematococcus pluvialis* NIES-144, the functions of the hydroxyl group-introducing enzyme gene (crtZ) from the non-photosynthetic *Erwinia uredovora* and minor ketocarotenoid biosynthesis pathways.

Fig. 10 shows two plasmids pHP5 and pHP51 each containing the keto group-introducing enzyme gene (bkt) from the green alga *Haematococcus pluvialis* NIES-144.

pHP5 is inserted into pSPORT I and pHP51 into pBluescript II KS⁺ in such a direction that they undergo the read-through of the lac promoter. The sites digested by restriction enzymes are abbreviated as follows: S, Sall; Ss, Sstl; P, Pstl; Sp, Sphl; N, Notl; X, Xbal; K. Kpnl; Sa, Sacl.

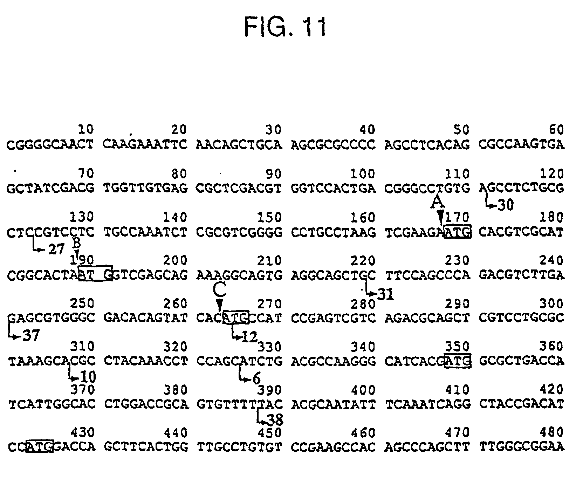

Fig. 11 shows the base sequence for a region including the initiation codons of the keto group-introducing enzyme gene (bkt) from the green alga *Haematococcus pluvialis* NIES-144 and indicates the initiation sites of various deletion plasmids.

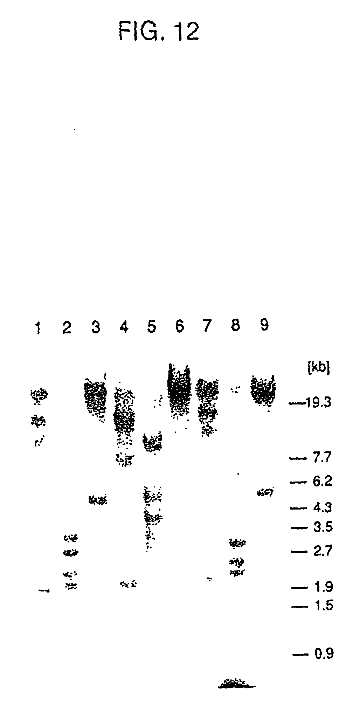

Fig. 12 shows the results of Southern analysis (electrophoresis photo) using as a probe a 1.7 kb DNA fragment of the green alga *Haematococcus pluvialis* NIES-144 bkt gene.
Lanes 1-3: *Haematococcus pluvialis* NIES-144
Lanes 4-6: *Haematococcus lacustris* UTEX294
Lanes 7-9: *Haematococcus lacustris* C392
Lanes 1, 4 and 7: HindIII digest
Lanes 2, 5 and 8: Pstl digest
Lanes 3, 6 and 9: Xbal digest

### BEST MODES FOR CARRYING OUT THE INVENTION

The present invention will be described more specifically below with reference to the following Examples, which should not be construed as limiting the scope of the invention.

### [Example 1] Biomaterials and the Medium Composition

The *Haematococcus pluvialis* used for obtaining genes is the NIES-144 strain registered at the Foundation Global Environmental Forum. *H. pluvialis* was cultured for 4 days in basal medium (yeast extract 0.2%; sodium acetate 0.12%; L-asparagine 0.04%; magnesium chloride - 6H₂O 0.02%; iron(II) sulfate . 7H₂O 0.001%; calcium chloride 2H₂O 0.002%) at 20°C under 12 hr light/12 hr dark cycles 1500 Lx (20 µE/m² · s). Further, forthe induction of astaxanthin synthesis in *H. pluvialis*, acetic acid was added to the *H. pluvialis* NIES-144 strain to a final concentration of 45 mM and iron(II) sulfate· 7H₂O to a final concentration of 450 µm, and the strain was cultured at 20°C at a photointensity of 9600 Lx (125 µE/m² · s) for about 12 hours to thereby induce the formation of cysts.

### [Example 2] Preparation of the Total DNA from Haematococcus pluvialis

The *H. pluvialis* NIES-144 strain was seeded on 400 ml of basal medium and cultured at 20 °C at a photointensity of 1500 Lx (20 µE/m² · s) under 12 hr light/12 hr dark cycles for about 4 days. Then, cells were harvested from the culture, frozen with liquefied nitrogen and crushed in a mortar until the cells became powder-like. To the powder-like cells, 15 ml of extraction buffer (0.1 M Tris-Hcl pH 8.0, 0.1 M EDTA, 0.25 M NaCl, 0.1 mg/ml Proteinase K) was added, stirred violently and then kept at 55°C for 2 hours. Then, the mixture was centrifuged at 6000xg for 10 minutes at 4°C to remove the precipitate. To the supernatant, 0.6 volume of isopropanol was added and cooled at -20°C for 30 minutes. Then, the mixture was centrifuged at 7500xg for 15 minutes at 4 °C. The centrifuged material containing DNA was dissolved in 2 ml of TE buffer (10 mM Tris-HCl pH 8.0, 1 mM EDTA), mixed with the same volume of phenol: chloroform (1:1) and then subjected to centrifugation to extract the upper layer. Subsequently, 80 µl of 5 M NaCl and 5 mL of ethanol were added to the upper layer, cooled at -20°C for 30 minutes and then centrifuged at 12000xg for 15 minutes at 4°C . The precipitate was rinsed with 70% ethanol and then dried. Thereafter, the precipitate was dissolved in 0.5 ml of TE buffer (10 mM Tris-HCl pH 8.0, 1 mM EDTA) and 2.5 µl of 10 mg/ml RNase A was added thereto to make a total DNA solution of *Haematococcus pluvialis.*

### [Example 3] Attempt to Isolate crtZ Homologous Regions from H. pluvialis by PCR

By comparing amino acid sequences encoded by crtZ genes from *Erwinia uredovora* and *Erwinia herbicola* (Misawa, N., Nakagawa, M., Kobayashi, K., Yamano, S., Izawa, Y., Nakamura, K. and Harashima, K., "Elucidation of the *Erwinia uredovora* carotenoid biosynthetic pathway by functional analysis of gene products expressed in *Escherichia coli*", J. Bacteriol., 172, pp. 6704-6712, 1990; Hundle, B.S., Beyer, P., Kleinig, H., Englert, G. and Hearst, J. E., "Carotenoids of *Erwinia herbicola* and an *Escherichia coli* HB101 strain carrying the *Erwinia herbicola* Carotenoid Gene Cluster", Phytochem. Phytobiol., 54, pp. 89-93, 1991), regions with a high homology were found out. By combining those codons which are expected to be used in view of the amino acid sequences of these regions, the following 3 primers were synthesized to prepare mixed primers.

No. 1 5'-GGNTGGGNTGGCAYAARTCNCAYCA-3'

No. 2 5'-CANCGYTGRTGNACNAGNCCRTCRTG-3'

No. 3 5'-GCRTASATRAANCCRAARCTNACRCA-3'

[N: A, G, C or T; R: A or G; Y: C or T; S: A, G or T]

A mixed primer consisting of No. 1 & No. 2 and another mixed primer consisting of No. 1 & No. 3 were prepared and PCR (polymerase chain reaction) was carried out using the total DNA solution of *H*. *pluvialis* as templates. The following materials were mixed so that they have the following final concentrations: about 100 ng total DNA solution of *H. pluvialis*; each 100 µm mixed primers; 1xVent Buffer [10 mM KCI, 20 mM Tris-HCl (pH 8.8), 10 mM (NH₄)₂SO₄, 2 mM MgSO₄, 0.1% Triton X-100); 250 µM dNTP; and 2 U Vent DNA polymerase (New England Biolabs, Inc.). The PCR was conducted 30 cycles with the conditions of at 94°C for 30 seconds, 55 °C for 30 seconds and 72°C for 30 seconds, and 30 cycles with the conditions of at 94 °C for 30 seconds, 60 °C for 30 seconds and 72°C for 30 seconds. Then, the presence of reaction products was confirmed by electrophoresis. However, in any of the cases, a definite, single product has not been detected.

### [Example 4] Preparation of the Total RNA from Haematococcus pluvialis

The *H. pluvialis* NIES-144 strain was seeded on 800 ml of basal medium and cultured at 20 °C at a photointensity of 1500 Lx (20 µE/m² · s) under 12 hr light/12 hr dark cycles for about 4 days. Then, acetic acid was added thereto to give a final concentration of 45 mM and iron(II) sulfate · 7H₂O to a final concentration of 450 µm. Thereafter, cells were cultured at 20°C at a photointensity of 9600 Lx (125 µE/m² · s) for about 12 hours. Then, cells were harvested from the culture, frozen with liquefied nitrogen and crushed in a mortar until the cells became powder-like. To the powder-like cells, 3 ml of ISOGEN-LS (Nippon Gene) was added and left at room temperature for 5 minutes. Further, 0.8 ml of chloroform was added thereto. The mixture was violently stirred for 15 seconds and then left at room temperature for 3 minutes. The resultant mixture was centrifuged at 12000xg for 15 minutes at 4°C to extract the upper layer. To the upper layer, 2 ml of isopropanol was added and left at room temperature for 10 minutes.

Then, the mixture was centrifuged at 12000xg for 10 minutes at 4°C . Subsequently, the precipitate was rinsed with 70% ethanol, dried and then dissolved in 1 ml of TE buffer (10 mM Tris-HCl pH 8.0, 1 mM EDTA) to obtain a total RNA solution of *Haematococcus pluvialis*. By the above procedures, 4.1 mg of the total RNA was obtained.

### [Example 5] Preparation of the cDNA Expression Library of Haematococcus pluvialis

Using Oligotex-dT30 Super™ (Takara Shuzo), poly A⁺ RNA was purified from approximately 1 mg of the total RNA of *H. pluvialis* according to the manufacture's protocol attached to the product. Approximately 14 µg of poly A⁺ mRNA was purified by this method.

cDNA was prepared by using Superscript TM® Plasmid System (Gibco BRL) according to the attached protocol with a partial modification as follows. By using approximately 5 µg of poly A⁺ RNA, a complementary DNA strand was synthesized with a synthetic DNA comprising the recognition sequence of the restriction enzyme Notl and an oligo-dT of 15-mers as a primer. Subsequently, by using *E. coli* DNA ligase, *E. coli* DNA polymerase and *E. coli* DNA RNase H, a double-stranded cDNA was synthesized. To this cDNA, the linker of the restriction enzyme Sail was ligated with T4 DNA ligase so that finally the upstream end of this cDNA would be a Sail site and the downstream of poly A an Notl site. The cDNAs obtained were fractionated by size by electrophoresis and the fractions containing fragments ranging from 0.7 kb to 3.5 kb were collected. About 28 ng of the cDNAs of these fractions and 35 ng of the cDNA expression vector pSPORT I (Gibco BRL) which was digested with Notl and Sail were ligated with the ligation buffer (50 mM Tris-HCl pH 7.6, 10 mM MgCl₂, 1 mM ATP, 1 mM DTT, 5% PEG 8000) contained in the above-described kit and T4 DNA ligase. This cDNA expression vector pSPORT I is a vector having a lac promoter upstream of a Sail site and capable of expressing a cDNA in *E. coli*. Then, using all of the ligated DNA solution, transformation of competent cells of the *E. coli* DH5_{α} which were prepared according to the method described in Molecular Cloning (2nd edition): Cold Spring Harbor Laboratory, 1.21-1.41 (1989) was carried out. About 40,000 strains of transformants were obtained. Collecting all of these transformants, plasmid DNA was'prepared according to the method described in Molecular Cloning (2nd edition): Cold Spring Harbor Laboratory, 1.21-1.41 (1989). As a result, 0.6 mg of plasmid DNA was obtained and this was made the cDNA expression library of *Haematococcus pluvialis .*

### [Example 6] Screening Utilizing the Changes of ColorTone in the Keto Group-Introducing Enzyme Gene Carrying E. coli

### (1) Preparation of β-carotene producing E. coli

By subjecting plasmid pCAR16 which contains all of the *Erwinia uredovora* carotenoid synthesis genes (crtE, crtX, crtY, crtl and crtB) other than crtZ (see Misawa, N., Nakagawa, M., Kobayashi, K., Yamano, S., Izawa, Y., Nakamura, K. and Harashima, K., "Elucidation of the *Erwinia uredovora* carotenoid biosynthetic pathway by functional analysis of gene products expressed in *Escherichia coli*", J. Bacteriol., 172, pp. 6704-6712, 1990; and Japanese Unexamined Patent Publication No. 3-58786) to BstEII digestion, Klenow enzyme treatment and a ligase reaction, the crtX gene was deactivated by a frameshift. Then, a 6.0 kb Asp718(KpnI)-EcoRI fragment was cut out which contains the crtE, crtY, crtl and crtB genes necessary for β -carotene production. This fragment was inserted into the EcoRV site of *E. coli* vector pACYC184 (obtained from ATCC 37033) to thereby obtain the plasmid of interest (designated as pACCAR16ΔcrtX). The *E. coli* carrying this pACCAR16ΔcrtX exhibits chloramphenicol resistance and can produce β -carotene.

### (2) Screening for the keto group-introducing enzyme gene

It is considered that ketocarotenoids are biosynthesized in *Haematococcus pluvialis* via β -carotene (see Britton, G., " Biosynthesis of carotenoids", Plant Pigments, Goodwin, W.W. (ed.), London, Academic Press, 1988, pp. 133-182). Then, utilizing the phenomenon that *E. coli* JM101 carrying the plasmid PACCAR16ΔcrtX described above produces β -carotene (yellow), the cDNA expression library obtained above was introduced to this *E. coli*. Subsequently, the *E. coli* carrying the keto group-introducing enzyme gene was screened from the color change in the resultant transformants. It was expected that, when keto groups were introduced and canthaxanthin (one of ketocarotenoids) began to be produced, the color of *E. coli* would change from the yellow of β -carotene to the red of canthaxanthin.

First, using the method described in Molecular Cloning (2nd edition): Cold Spring Harbor Laboratory, 1.21-1.41 (1989), competent cells of *E. coli* JM101 carrying pACCAR16ΔcrtX were prepared.

Then, to 1 ml of these competent cells, 100 ng of the cDNA expression library was introduced, and the screening was conducted for about 40,000 transformants to thereby isolate one strain which was reddish and slightly different from others in color tone. (The pigment of this strain was identified as canthaxanthin in Example 7.) In addition, the cDNA expression plasmid carried by this strain was designated as pHP5. The constitution of plasmid pHP5 is shown in Fig. 10.

### [Example 7] Determination of the Base Sequence of the Keto Group-Introducing Enzyme Gene

A *Haematococcus pluvialis*-derived 1.7 kb cDNA inserted into pPH5 was cut out with the restriction enzymes Sall and Xbal. This fragment was inserted into the Sall/Xbal site of both *E. coli* vector pBluescript II KS⁺ and *E. coli* vector pBluescript II® SK⁺ to thereby obtain two plasmids (pHP51 and pHP52). Of these plasmids, the restriction map of pHP51 is shown in Fig. 10. pHP51 and pHP52 are different in the direction of the above cDNA fragment inserted therein. In the former plasmid, the cDNA fragment undergoes the read-through of the lac promoter and in the latter the cDNA fragment does not.

Using the obtained plasmids pHP51 and pHP52, deletion variants having various lengths of deletions were prepared by the following procedures and their base sequences were determined. pHP51 was digested with Sacl and Xbal, and pHP52 with KpnI and Sall. Then, phenol/chloroform extraction was carried out and the DNA was recovered by ethanol precipitation. Each DNA was dissolved in 100 µl of Exolll buffer (50 mM Tris-HCl, 100 mM NaCl, 5 mM MgCl₂, 10 mM 2-mercaptoethanol, pH 8.0) and, after the addition of 180 units of Exolll nuclease thereto, kept at 37 °C. By sampling a 10 µl reaction solution in every 30 seconds, each sample was transfered into a tube containing 10 µl of MB buffer (40 mM NaCl, 2 mM ZnCl₂, 10% glycerol, pH 4.5) located on ice. After the completion of the sampling, the 10 tubes obtained was kept at 65°C for 10 minutes to deactivate enzymes. Then, 5 units of mung bean nuclease was added thereto and kept at 37°C for 30 minutes. After the completion of the reaction, 10 kinds of DNA fragments having varying degrees of deletions were recovered per one plasmid by agarose gel electrophoresis. The recovered DNAs were blunt-ended with Klenow enzyme and subjected to ligation reaction at 16 °C overnight, to thereby transform *E. coli* DH5_{α}. Plasmids were prepared for the resultant various clones, and sequence reactions were performed by using a fluorescent primer cycle sequence kit manufactured by Applied Biosystems. Then, the base sequence of each plasmid was determined with an automatic sequencer.

The thus determined base sequence consisting of 1677 bp is shown in Figs. 4 and 5 (SEQ ID NO: 4). As a result of search for open reading frames, 3 open reading frames have been found which individually have a ribosome binding site at the upstream of the initiation codon that is necessary for the expression in *E. coli.* These three frames are shown individually as A-D in Fig. 1 (SEQ ID NO: 5 in the sequence listing), as B-D in Fig. 2 (SEQ ID NO: 6) and as C-D in Fig. 3 (SEQ ID NO: 7). As demonstrated in Example 8 *infra*, a shorter polypeptide than C-D loses the enzyme activity in *E. coli ,* and thus it is considered that no initiation codon exists downstream of C. Therefore, the region locating downstream of C in Fig. 3 was excluded from the search for open reading frames as described above.

### [Example 8] Determination of the Initiation Codon for the Keto Group-Introducing Enzyme Gene

Fig. 11 shows the base sequence for an upstream portion of the open reading frames described above. There are 5 potential initiation codon sites (base positions 168-170, 189-191, 264-266, 348-350 and 423-425; these sites are enclosed with boxes in Fig. 11). The bases at positions 168, 189 and 264 shown in the initiation codons in Fig. 11 correspond to positions A in Fig. 1, B in Fig. 2 and C in Fig. 3, respectively. In order to determine the necessary minimum region as a functional protein, deletion variants of pHP51 were prepared in substantially the same manner as in Example 5, to thereby obtain several plasmids wherein the upstream region was deleted. Fig. 11 shows the number of each of these deletion plasmids and their upstream ends. These plasmids were individually introduced into the *E. coli* JM101 carrying pACCAR16ΔcrtX as described in Example 6 and the pigments produced were identified. As a result, *E. coli* cells carrying deletion plasmids Nos. 30, 27, 31, 37 and 12 were observed to produce canthaxanthin, but those cells carrying deletion plasmids Nos. 10, 6 and 38 were not observed to produce it. With respect to the deletion plasmid No. 12 which lacks A of the initiation codon ATG at base positions 264-266, this ATG became GTG when a deletion variant was produced. Since *E. coli* can recognize even GTG as an initiation codon, it is considered that the synthesis of a peptide is starting from the initiation codon at this position. Therefore, it has become clear that a polypeptide chain encoded by the open reading frame starting from the initiation codon at positions 264-266 [C-D in Fig. 3 (as shown in SEQ ID NO: 7)] sufficiently exhibits the enzyme activity of keto group introduction.

### [Example 9] Identification of a Ketocarotenoid Pigment

### (1) Identification of canthaxanthin

β-carotene producing *E. coli* JM101 into which pHP5 or pHP51 has been introduced (*E. coli* pACCAR16ΔcrtX, pHP5 or pHP51) (presenting an orange color) was cultured in 2 liters of 2YT medium (1.6% tryptone, 1% yeast extract, 0.5% NaCl) containing 150 µg/ml ampicillin (Ap, Meiji Seika Kaisha), 30 µg/ml chloramphenicol (Cm, Sankyo, Co.), 1 mM IPTG, 7 mg FeSO₄ · 7H₂O and 9.3 mg Na₂ · EDTA at 30°C for 24-30 hours. Cells harvested from the culture were extracted with 300 ml of acetone, and after concentration, extracted twice with 200 ml of chloroform/methanol (9/1) followed by concentration/drying/caking. The resultant material was dissolved in a small amount of chloroform/methanol (9/1) and subjected to thin layer chromatography (TLC) using a preparatory silica gel plate (Merck) and developing with chloroform/methanol (50/1). By means of this TLC, spots were separated into three with Rf values of 0.53, 0.78 and 1. The most dark red pigment (of Rf value 0.53) representing 75% of the total pigments extracted was recovered from the TLC plate. This red pigment was further dissolved in a small amount of chloroform/methanol (9/1), applied to Sephadex LH-20 column chromatography (15 x 300 mm) and developed and eluted with chloroform/methanol (9/1) or chloroform/methanol (1/1), to thereby obtain 2 mg of a pure pigment. All of the ultraviolet-visible spectrum, ¹H-NMR, FD-MS spectrum (m/e 564) and mobility on silica gel TLC [the Rf value was 0.53 when developed with chloroform/methanol (50/1)] of this substance agreed with those of a standard canthaxanthin product (BASF), and thus this substance was identified as canthaxanthin (for the structural formula, see Fig. 8).

Further, a red pigment (having an Rf value of 0.78 on TLC) which represented 10% of the total pigments initially extracted was recovered from the TLC plate and dissolved in a small amount of methanol. In view of the ultraviolet-visible spectrum, mobility on silica gel TLC [the Rf value was 0.78 when developed with chloroform/methanol (50/1)] and mobility on HPLC using Novapack® HR6 µ C₁₈ (3.9 x 300 mm) (Waters) [RT was 16 minutes when developed with acetonitrile/methanol/2-propanol /2-propanol (90/6/4) at a flow rate of 1.0 ml/min] of this pigment, it was believed to be echinenone (for the structural formula, see Fig. 8).

Then, a yellow pigment (having an Rf value of 1 on TLC) which represented the remaining 15% of the total pigments initially extracted was scraped from the TLC plate and dissolved in a small amount of methanol. Since the ultraviolet-visible spectrum and mobility on HPLC using Novapack HR6 µ C₁₈ (3.9 x 300 mm) (Waters) [RT was 62 minutes when developed with acetonitrile/methanol/2-propanol (90/6/4) at a flow rate of 1.0 ml/mon] of this pigment agreed with those of a β -carotene standard product (all trans type, Sigma), this substance was found to be an unreacted β -carotene (for the structural formula, see Fig. 8).

### (2) Identification of astaxanthin and 4-ketozeaxanthin

A zeaxanthin-producing *E. coli* was prepared as follows. Briefly, plasmid pCAR25 having all of the carotenoid synthesis genes from *Er*. *uredovora* (Misawa, N., Nakagawa, M., Kobayashi, K., Yamano, S., Izawa, Y., Nakamura, K. and Harashima, K., "Elucidation of the *Erwinia uredovora* carotenoid biosynthetic pathway by functional analysis of gene products expressed in *Escherichia coli*", J. Bacteriol., 172, pp. 6704-6712, 1990; and Japanese Unexamined Patent Publication No. 3-58786) was subjected to BstEII digestion, Klenow fragment treatment and a ligase reaction to thereby deactivate the crtX gene by a frameshift. Then, a 6.5 kb Asp718(KpnI)-EcoRI fragment was cut out which contains the crtE, crtB, crt, crtY and crtZ genes necessary for zeaxanthin production. This fragment was inserted into the EcoRV site of *E. coli* vector pACYC184 to thereby obtain the plasmid of interest (designated as PACCAR25ΔCrtX).

The zeaxanthin-producing *E. coli* JM101 into which pHP5 or pHP51 has been introduced (*E. coli* pACCAR25ΔcrtX, pHP5 or pHP51) (presenting an orange color) was cultured in 2 liters of 2YT medium (1.6% tryptone, 1% yeast extract, 0.5% NaCl) containing 150 µg/ml Ap, 30 µg/ml Cm, 1 mM IPTG, 7 mg FeSO₄ · 7H₂O and 9.3 mg Na₄ EDTA at 30°C for 24-30 hours. Cells harvested from the culture were extracted with 300 ml of acetone, and after concentration, extracted twice with 200 ml of chloroform/methanol (9/1) followed by concentration/drying/caking. The resultant material was dissolved in a small amount of chloroform/methanol (9/1) and subjected to thin layer chromatography (TLC) using a preparatory silica gel TLC plate (Merck) and developing with chloroform/methanol (15/1). By means of this TLC, the initial orange pigment was separated into 3 major spots with Rf values of 0.40, 0.54 and 0.72. These pigments were recovered from the TLC plate, dissolved separately in a small amount of chloroform/methanol (9/1), applied to Sephadex LH-20 column chromatography (15 x 300 mm) and developed and eluted with chloroform/methanol (9/1) or methanol, to thereby obtain three pure pigments in amounts of about 1 mg, 1 mg and 2 mg.

A pigment having an Rf value of 0.72 which represented about half of the total pigments extracted was found to have the same planar structure as that of astaxanthin in view of the results of its ultraviolet-visible spectrum, ¹H-NMR and FD-MS spectrum (m/e 596). Then, this pigment was dissolved in diethyl ether:2-propanol:ethanol (5:5:2) and measured the CD spectrum. As a result, this substance was found to take a steric structure of 3S,3'S. Therefore, this substance was identified as astaxanthin (for the structural formula, see Fig. 8). Another pigment of Rf 0.54 was identified as 4-ketozeaxanthin (for the structural formula, see Fig. 8) in view of the results of its ultraviolet-visible spectrum, ¹H-NMR, FD-MS spectrum (m/e 582) and mobility on silica gel TLC [the Rf value was 0.54 when developed with chloroform/methanol (15/1)]. With respect to the pigment having an Rf value of 0.40, its ultraviolet-visible spectrum, mobility on silica gel TLC [the Rf value was 0.40 when developed with chloroform/methanol (50/1)] and mobility on HPLC using Novapack® HR6 µ C₁₈ (3.9 x 300 mm) (Waters) [RT was 6.5 minutes when developed with acetonitrile/methano 1/2-propanol (90/6/4) at a flow rate of 1.0 ml/min] all agreed with those of a zeaxanthin standard product (BASF). Therefore, this substance was found to be an unreacted zeaxanthin (for the structural formula, see Fig. 8).

From so far described, the functions of the keto group-introducing enzyme gene can be considered as follows.

From (1) of Example 9, it is clear that the *Haematococcus*-derived keto group-introducing enzyme gene (bkt) is coding for a keto group-introducing enzyme (β -carotene ketolase) which catalyzes the conversion of β -carotene (a substrate) to canthaxanthin via echinenone (see Fig. 8). This shows that one enzyme, BKT, converts the methylene groups at positions 4 and 4'of a β -ionone ring directly to keto groups. No enzyme having such a function has been known so far. In addition, from (2) of Example 9, it is clear that the *Haematococcus*-derived bkt gene is also coding for another keto group-introducing enzyme (zeaxanthin ketolase) which catalyzes the conversion of zeaxanthin (a substrate) to astaxanthin via 4-ketozeaxanthin (see Fig. 8). This shows that one enzyme, BKT, converts the methylene groups at positions 4 and 4'of 3- and 3'-hydroxy-β -ionone rings directly to keto groups. No enzyme having such a function has been known so far neither. Accordingly, it can be said that the *Haematococcus*-derived keto group-introducing enzyme gene bkt is coding for an β-ionone or 3-hydroxy-β-ionone -ionone ring keto group-introducing enzyme β -ionone or 3-hydroxy-β-ionone -ionone ring ketolase) which converts the methylene group at position 4 (4') to a keto group directly, regardless of whether a hydroxyl group is added to position 3 (3'). Not limited in β -ionone rings or 3-hydroxy-β -ionone rings, there has been reported no finding so far that one enzyme converts a methylene group to a keto group directly.

On the other hand, according to the researches of the present inventors using carotenoid synthesis genes from the bacteria *Erwinia* present in plants and the photosynthetic bacteria *Rhodobacter*, it has become clear that, generally, a carotenoid biosynthesis enzyme recognizes only one half of the carotenoid molecule which is a substrate and acts on it. For example, crtY which is a lycopene ring formation enzyme gene recognizes by one half of the lycopene molecule and makes the ring formation. Therefore, by using the phytoene desaturase gene crtl from *Rhodobacter*, it is possible to allow *E. coli* to produce neurosporene instead of lycopene. And when the produced neurosporene is treated with the *Erwinia*-derived crtY, the crtY gene product recognizes only the half structure of a neurosporene molecule which is common with lycopene and, as a result, -zeacarotene is produced which is circulized by half (see Linden, H., Misawa, N., Chamovitz, D., Pecker, I., Hirschberg, J. and Sandmann, G., "Functional complementation in *Escherichia coli* of different phytoene desaturase genes and analysis of accumulated carotenes", Z. Naturforsch., 46c, pp. 1045-1051, 1991). In addition, in the present invention also, when β -carotene was treated with BKT, first echinenone is synthesized wherein one keto group is introduced, and when zeaxanthin is treated with BKT, first 4-ketozeaxanthin is synthesized wherein one keto group is introduced. This can be considered that BKT recognizes one half of a substrate molecule and introduces a keto group at position 4. On the other hand, the *E. coli* carrying the *Erwinia*-derived crtE, crtB, crtl, crtY and crtZ genes produces zeaxanthin as described above, but β-cryptoxanthin wherein one hydroxyl group is introduced into β-carotene can also be detected in the products as an intermediary metabolite. This means that, if BKT is present there, 3'-hydroxyechinenone and 3-hydroxyech inenone can be produced with the β-cryptoxanthin as a substrate. In addition, it can be also considered that BKT further acts on these substances produced to thereby synthesize phoenicoxanthin. This time, we have not achieved the identification of these substances in cultures, because under the conditions employed for this time it seems that these substances are present only in extremely small amounts. In fact, in the typical astaxanthin-producing microorganism *Phaffia rhodozyma* which is comparable with *Haematococcus,* 3-hydroxyechinenone and phoenicoxanthin are detected as intermediary metabolites of astaxanthin (Andrewes, A. G., Phaff, H. J. and Starr, M. P., "Carotenoids of *Phaffia rhodozyma*, a red-pigmented fermenting yeast", Phytochemistry, 15, pp. 1003-1007, 1976). From so far described, it is possible to consider that there are the minor metabolic pathways shown in Fig. 9 other than the major astaxanthin metabolic pathway shown in Fig. 8.

### [Example 10] Southern Analysis of the Genomic DNA of the other Green Algae Haematococcus

It was examined as to whether some regions showing homology with bkt's isolated in the chromosomes of the other green algae *Haematococcus*. In the same manner as described in Example 2 for preparing the total DNA of *Haematococcus pluvialis* NIES-144, the total DNAs of *Haematococcus lucustris* UTEX 294 and *Haematococcus lucustris* C-392 were prepared. The resultant DNAs together with the total DNA of *H. pluvialis* NIES-144 were digested with the restriction enzyme HindIII, Pstl or Xbal and separated by agarose gel electrophoresis. The separated DNA fragments were denatured with an alkali solution of 0.5 N NaOH/1.5 M NaCl, and then transferred to a nylon membrane overnight. The nylon membrane which had adsorbed DNA was soaked in a hybridization solution (6x Denhardt, 5xSSC, 0.2% SDS, 100 µg/ml ssDNA) to carry out a prehybridization for 4 hours at 55 °C. Then, a 1.7 kb DNA fragment of bkt gene was labelled by using Megaprime™ DNA labelling system (Amersham) and [_{α}-³²P]dCTP (up to 110 TBq/mmol) and added to the prehybridization solution described above to thereby carry out a hybridization for 16 hours at 55 °C . After the hybridization, the reaction solution was washed with 2xSSC and 0.1% SDS at 60 °C for 1 hour and subjected to autoradiography to detect signals indicating homology. As a result, with respect to *Haematococcus pluvialis* NIES-144, strong signals were obtained at positions 15kb, 10 kb and 1.9 kb in HindIII digest, 6.1 kb, 3.3 kb, 2.8 kb, 2.3 kb, 2.0 kb, 1.4 kb and 0.8 kb in Pstl digest and 5.1 kb in Xbal digest. With respect to *Haematococcus lucustris* UTEX 294, strong signals were obtained at positions 15kb, 7.7 kb and 1.9 kb in HindIII digest, 10 kb, 5.0 kb, 4.0 kb, 3.4 kb, 2.9 kb, 1.5 kb and 0.82 kb in Pstl digest and only at a position more than 20 kb in Xbal digest. With respect to *Haematococcus lucustris* C-392, strong signals were obtained at positions 15kb, 12 kb and 1.9 kb in HindIII digest, 6.5 kb, 3.0 kb, 2.3 kb, 2.0 kb, 1.4 kb and 0.8 kb in Pstl digest and 5. 3 kb in Xbal digest (see Fig. 12).

### INDUSTRIAL APPLICABILITY

By introducing into a microorganism such as *E. coli* as a foreign gene the DNA of the invention coding for an enzyme which convert the methylene group at position 4 of β -ionone ring to a keto group and allowing the microorganism to express the DNA, it has become possible to render a microorganism such as *E. coli* an ability to biosynthesize ketocarotenoids such as astaxanthin, 4-ketozeaxanthin, canthaxanthin, echinenone and other keto group-containing ketocarotenoids. By using the microorganism such as *E. coli* which has been rendered the ability to biosynthesize keto group-containing ketocarotenoids, it is possible to produce keto group-containing ketocarotenoids in large quantity with small labor and at low cost.

### SEQUENCE LISTING

### INFORMATION FOR SEQ ID NO: 1

### SEQUENCE CHARACTERISTICS:

LENGTH: 320 amino acids

TYPE: amino acids

TOPOLOGY: linear

### MOLECULAR TYPE: peptide

### SOURCE:

SPECIES: *Haematococcus pluvialis*

STRAIN: NIES-144

### SEQUENCE DESCRIPTION: SEQ ID NO: 1:

Met His Val Ala Ser Ala Leu Met Val Glu Gln Lys Gly Ser Glu
1               5                   10                  15
Ala Ala Ala Ser Ser Pro Asp Val Leu Arg Ala Trp Ala Thr Gln
                20                  25                  30
Tyr His Met Pro Ser Glu Ser Ser Asp Ala Ala Arg Pro Ala Leu
                35                  40                  45
Lys His Ala Tyr Lys Pro Pro Ala Ser Asp Ala Lys Gly Ile Thr
                50                  55                  60
Met Ala Leu Thr Ile Ile Gly Thr Trp Thr Ala Val Phe Leu His
                65                  70                  75
Ala Ile Phe Gln Ile Arg Leu Pro Thr Ser Met Asp Gln Leu His
                80                  85                  90
Trp Leu Pro Val Ser Glu Ala Thr Ala Gln Leu Leu Gly Gly Ser
                95                  100                 105
Ser Ser Leu Leu His Ile Ala Ala Val Phe Ile Val Leu Glu Phe
                110                 115                 120

Leu Tyr Thr Gly Leu Phe Ile Thr Thr His Asp Ala Met His Gly
                125                 130                 135
Thr Ile Ala Leu Arg His Arg Gln Leu Asn Asp Leu Leu Gly Asn
                140                 145                 150
Ile Cys Ile Ser Leu Tyr Ala Trp Phe Asp Tyr Ser Met Leu His
                155                 160                 165
Arg Lys His Trp Glu His His Asn His Thr Gly Glu Val Gly Lys
                170                 175                 180
Asp Pro Asp Phe His Lys Gly Asn Pro Gly Leu Val Pro Trp Phe
                185                 190                 195
Ala Ser Phe Met Ser Ser Tyr Met Ser Leu Trp Gln Phe Ala Arg
                200                 205                 210
Leu Ala Trp Trp Ala Val Val Met Gln Met Leu Gly Ala Pro Met
                215                 220                 225
Ala Asn Leu Leu Val Phe Met Ala Ala Ala Pro Ile Leu Ser Ala
                230                 235                 240
Phe Arg Leu Phe Tyr Phe Gly Thr Tyr Leu Pro His Lys Pro Glu
                245                 250                 255
Pro Gly Pro Ala Ala Gly Ser Gln Val Met Ala Trp Phe Arg Ala
                260                 265                 270
Lys Thr Ser Glu Ala Ser Asp Val Met Ser Phe Leu Thr Cys Tyr
                275                 280                 285
His Phe Asp Leu His Trp Glu His His Arg Trp Pro Phe Ala Pro
                290                 295                 300
Trp Trp Gln Leu Pro His Cys Arg Arg Leu Ser Gly Arg Gly Leu
                305                 310                 315
Val Pro Ala Leu Ala
                320

### INFORMATION FOR SEQ ID NO: 2

### SEQUENCE CHARACTERISTICS:

LENGTH: 313 amino acids

TYPE: amino acids

TOPOLOGY: linear

### MOLECULAR TYPE: peptide

### SOURCE:

SPECIES: *Haematococcus pluvialis*

STRAIN: NIES-144

### SEQUENCE DESCRIPTION: SEQ ID NO: 2:

Met Val Glu Gln Lys Gly Ser Glu Ala Ala Ala Ser Ser Pro Asp
1               5                   10                  15
Val Leu Arg Ala Trp Ala Thr Gln Tyr His Met Pro Ser Glu Ser
                20                  25                  30
Ser Asp Ala Ala Arg Pro Ala Leu Lys His Ala Tyr Lys Pro Pro
                35                  40                  45
Ala Ser Asp Ala Lys Gly Ile Thr Met Ala Leu Thr Ile Ile Gly
                50                  55                  60
Thr Trp Thr Ala Val Phe Leu His Ala Ile Phe Gln Ile Arg Leu
                65                  70                  75
Pro Thr Ser Met Asp Gln Leu His Trp Leu Pro Val Ser Glu Ala
                80                  85                  90
Thr Ala Gln Leu Leu Gly Gly Ser Ser Ser Leu Leu His Ile Ala
                95                  100                 105
Ala Val Phe Ile Val Leu Glu Phe Leu Tyr Thr Gly Leu Phe Ile
                110                 115                 120
Thr Thr His Asp Ala Met His Gly Thr Ile Ala Leu Arg His Arg
                125                 130                 135

Gln Leu Asn Asp Leu Leu Gly Asn Ile Cys Ile Ser Leu Tyr Ala
                140                 145                 150
Trp Phe Asp Tyr Ser Met Leu His Arg Lys His Trp Glu His His
                155                 160                 165
Asn His Thr Gly Glu Val Gly Lys Asp Pro Asp Phe His Lys Gly
                170                 175                 180
Asn Pro Gly Leu Val Pro Trp Phe Ala Ser Phe Met Ser Ser Tyr
                185                 190                 195
Met Ser Leu Trp Gln Phe Ala Arg Leu Ala Trp Trp Ala Val Val
                200                 205                 210
Met Gln Met Leu Gly Ala Pro Met Ala Asn Leu Leu Val Phe Met
                215                 220                 225
Ala Ala Ala Pro Ile Leu Ser Ala Phe Arg Leu Phe Tyr Phe Gly
                230                 235                 240
Thr Tyr Leu Pro His Lys Pro Glu Pro Gly Pro Ala Ala Gly Ser
                245                 250                 255
Gln Val Met Ala Trp Phe Arg Ala Lys Thr Ser Glu Ala Ser Asp
                260                 265                 270
Val Met Ser Phe Leu Thr Cys Tyr His Phe Asp Leu His Trp Glu
                275                 280                 285
His His Arg Trp Pro Phe Ala Pro Trp Trp Gln Leu Pro His Cys
                290                 295                 300
Arg Arg Leu Ser Gly Arg Gly Leu Val Pro Ala Leu Ala
305 310 313

### INFORMATION FOR SEQ ID NO: 3

### SEQUENCE CHARACTERISTICS:

LENGTH: 288 amino acids

TYPE: amino acids

TOPOLOGY: linear

### MOLECULAR TYPE-: peptide

### SOURCE:

SPECIES: *Haematococcus pluvialis*

STRAIN: NIES-144

### SEQUENCE DESCRIPTION: SEQ ID NO: 3:

Met Pro Ser Glu Ser Ser Asp Ala Ala Arg Pro Ala Leu Lys His
1               5                   10                  15
Ala Tyr Lys Pro Pro Ala Ser Asp Ala Lys Gly Ile Thr Met Ala
                20                  25                  30
Leu Thr Ile Ile Gly Thr Trp Thr Ala Val Phe Leu His Ala Ile
                35                  40                  45
Phe Gln Ile Arg Leu Pro Thr Ser Met Asp Gln Leu His Trp Leu
                50                  55                  60
Pro Val Ser Glu Ala Thr Ala Gln Leu Leu Gly Gly Ser Ser Ser
                65                  70                  75
Leu Leu His Ile Ala Ala Val Phe Ile Val Leu Glu Phe Leu Tyr
                80                  85                  90
Thr Gly Leu Phe Ile Thr Thr His Asp Ala Met His Gly Thr Ile
                95                  100                 105
Ala Leu Arg His Arg Gln Leu Asn Asp Leu Leu Gly Asn Ile Cys
                110                 115                 120
Ile Ser Leu Tyr Ala Trp Phe Asp Tyr Ser Met Leu His Arg Lys
                125                 130                 135
His Trp Glu His His Asn His Thr Gly Glu Val Gly Lys Asp Pro
                140                 145                 150
Asp Phe His Lys Gly Asn Pro Gly Leu Val Pro Trp Phe Ala Ser
                155                 160                 165

Phe Met Ser Ser Tyr Met Ser Leu Trp Gln Phe Ala Arg Leu Ala
                170                 175                 180
Trp Trp Ala Val Val Met Gln Met Leu Gly Ala Pro Met Ala Asn
                185                 190                 195
Leu Leu Val Phe Met Ala Ala Ala Pro Ile Leu Ser Ala Phe Arg
                200                 205                 210
Leu Phe Tyr Phe Gly Thr Tyr Leu Pro His Lys Pro Glu Pro Gly
                215                 220                 225
Pro Ala Ala Gly Ser Gln Val Met Ala Trp Phe Arg Ala Lys Thr
                230                 235                 240
Ser Glu Ala Ser Asp Val Met Ser Phe Leu Thr Cys Tyr His Phe
                245                 250                 255
Asp Leu His Trp Glu His His Arg Trp Pro Phe Ala Pro Trp Trp
                260                 265                 270
Gln Leu Pro His Cys Arg Arg Leu Ser Gly Arg Gly Leu Val Pro
                275                 280                 285
Ala Leu Ala
288

### INFORMATION FOR SEQ ID NO: 4

### SEQUENCE CHARACTERISTICS:

LENGTH: 1677 base pairs

TYPE: nucleic acids

STRANDEDNESS: double

TOPOLOGY: linear

### MOLECULAR TYPE: cDNA

### SOURCE:

SPECIES: *Haematococcus Pluvialis*

STRAIN: NIES-144

### SEQUENCE DESCRIPTION: SEQ ID NO: 4:

CGGGGCAACT CAAGAAATTC AACAGCTGCA AGCGCGCCCC AGCCTCACAG CGCCAAGTGA 60
GCTATCGACG TGGTTGTGAG CGCTCGACGT GGTCCACTGA CGGGCCTGTG AGCCTCTGCG 120
CTCCGTCCTC TGCCAAATCT CGCGTCGGGG CCTGCCTAAG TCGAAGAATG CAC GTC 176
Met His Val
1
GCA TCG GCA CTA ATG GTC GAG CAG AAA GGC AGT GAG GCA GCT GCT TCC 224
Ala Ser Ala Leu Met Val Glu Gln Lys Gly Ser Glu Ala Ala Ala Ser
    5                   10                  15
AGC CCA GAC GTC TTG AGA GCG TGG GCG ACA CAG TAT CAC ATG CCA TCC 272
Ser Pro Asp Val Leu Arg Ala Trp Ala Thr Gln Tyr His Met Pro Ser
20                  25                  30                  35
GAG TCG TCA GAC GCA GCT CGT CCT GCG CTA AAG CAC GCC TAC AAA CCT 320
Glu Ser Ser Asp Ala Ala Arg Pro Ala Leu Lys His Ala Tyr Lys Pro
                40                  45                  50
CCA GCA TCT GAC GCC AAG GGC ATC ACG ATG GCG CTG ACC ATC ATT GGC 368
Pro Ala Ser Asp Ala Lys Gly Ile Thr Met Ala Leu Thr Ile Ile Gly
            55                  60                  65
ACC TGG ACC GCA GTG TTT TTA CAC GCA ATA TTT CAA ATC AGG CTA CCG 416
Thr Trp Thr Ala Val Phe Leu His Ala Ile Phe Gln Ile Arg Leu Pro
        70                  75                  80
ACA TCC ATG GAC CAG CTT CAC TGG TTG CCT GTG TCC GAA GCC ACA GCC 464
Thr Ser Met Asp Gln Leu His Trp Leu Pro Val Ser Glu Ala Thr Ala
    85                  90                  95
CAG CTT TTG GGC GGA AGC AGC AGC CTA CTG CAC ATC GCT GCA GTC TTC 512
Gln Leu Leu Gly Gly Ser Ser Ser Leu Leu His Ile Ala Ala Val Phe
100                 105                 110                 115
ATT GTA CTT GAG TTC CTG TAC ACT GGT CTA TTC ATC ACC ACA CAT GAC 560

Ile Val Leu Glu Phe Leu Tyr Thr Gly Leu Phe Ile Thr Thr His Asp
                120                 125                 130
GCA ATG CAT GGC ACC ATA GCT TTG AGG CAC AGG CAG CTC AAT GAT CTC 608
Ala Met His Gly Thr Ile Ala Leu Arg His Arg Gln Leu Asn Asp Leu
            135                 140                 145
CTT GGC AAC ATC TGC ATA TCA CTG TAC GCC TGG TTT GAC TAC AGC ATG 656
Leu Gly Asn Ile Cys Ile Ser Leu Tyr Ala Trp Phe Asp Tyr Ser Met
        150                 155                 160
CTG CAT CGC AAG CAC TGG GAG CAC CAC AAC CAT ACT GGC GAA GTG GGG 704
Leu His Arg Lys His Trp Glu His His Asn His Thr Gly Glu Val Gly
    165                 170                 175
AAA GAC CCT GAC TTC CAC AAG GGA AAT CCC GGC CTT GTC CCC TGG TTC 752
Lys Asp Pro Asp Phe His Lys Gly Asn Pro Gly Leu Val Pro Trp Phe
180                 185                 190                 195
GCC AGC TTC ATG TCC AGC TAC ATG TCC CTG TGG CAG TTT GCC CGG CTG 800
Ala Ser Phe Met Ser Ser Tyr Met Ser Leu Trp Gln Phe Ala Arg Leu
                200                 205                 210
GCA TGG TGG GCA GTG GTG ATG CAA ATG CTG GGG GCG CCC ATG GCA AAT 848
Ala Trp Trp Ala Val Val Met Gln Met Leu Gly Ala Pro Met Ala Asn
            215                 220                 225
CTC CTA GTC TTC ATG GCT GCA GCC CCA ATC TTG TCA GCA TTC CGC CTC 896
Leu Leu Val Phe Met Ala Ala Ala Pro Ile Leu Ser Ala Phe Arg Leu
        230                 235                 240
TTC TAC TTC GGC ACT TAC CTG CCA CAC AAG CCT GAG CCA GGC CCT GCA 944
Phe Tyr Phe Gly Thr Tyr Leu Pro His Lys Pro Glu Pro Gly Pro Ala
    245                 250                 255
GCA GGC TCT CAG GTG ATG GCC TGG TTC AGG GCC AAG ACA AGT GAG GCA 992
Ala Gly Ser Gln Val Met Ala Trp Phe Arg Ala Lys Thr Ser Glu Ala
260                 265                 270                 275

TCT GAT GTG ATG AGT TTC CTG ACA TGC TAC CAC TTT GAC CTG CAC TGG 1040
Ser Asp Val Met Ser Phe Leu Thr Cys Tyr His Phe Asp Leu His Trp
                280                 285                 290
GAG CAC CAC AGG TGG CCC TTT GCC CCC TGG TGG CAG CTG CCC CAC TGC 1088
Glu His His Arg Trp Pro Phe Ala Pro Trp Trp Gln Leu Pro His Cys
            295                 300                 305
CGC CGC CTG TCC GGG CGT GGC CTG GTG CCT GCC TTG GCA TGACCTGGTC 1137
Arg Arg Leu Ser Gly Arg Gly Leu Val Pro Ala Leu Ala
        310                 315                 320
CCTCCGCTGG TGACCCAGCG TCTGCACAAG AGTGTCATGC TACAGGGTGC TGCGGCCAGT 1197
GGCAGCGCAG TGCACTCTCA GCCTGTATGG GGCTACCGCT GTGCCACTGA GCACTGGGCA 1257
TGCCACTGAG CACTGGGCGT GCTACTGAGC AATGGGCGTG CTACTGAGCA ATGGGCGTGC 1317
TACTGACAAT GGGCGTGCTA CTGGGGTCTG GCAGTGGCTA GGATGGAGTT TGATGCATTC 1377
AGTAGCGGTG GCCAACGTCA TGTGGATGGT GGAAGTGCTG AGGGGTTTAG GCAGCCGGCA 1437
TTTGAGAGGG CTAAGTTATA AATCGCATGC TGCTCATGCG CACATATCTG CACACAGCCA 1497
GGGAAATCCC TTCGAGAGTG ATTATGGGAC ACTTGTATTG GTTTCGTGCT ATTGTTTTAT 1557
TCAGCAGCAG TACTTAGTGA GGGTGAGAGC AGGGTGGTGA GAGTGGAGTG AGTGAGTATG 1617
AACCTGGTCA GCGAGGTGAA CAGCCTGTAA TGAATGACTC TGTCTAAAAA AAAAAAAAAA 1677

### INFORMATION FOR SEQ ID NO: 5

### SEQUENCE CHARACTERISTICS:

LENGTH: 963 base pairs

TYPE: nucleic acids

STRANDEDNESS: double

TOPOLOGY: linear

### MOLECULAR TYPE: CDNA

### SOURCE:

SPECIES: *Haematococcus pluvialis*

STRAIN: NIES-144

### SEQUENCE DESCRIPTION: SEQ ID NO: 5:

ATG CAC GTC GCA TCG GCA CTA ATG GTC GAG CAG AAA GGC AGT GAG 45
Met His Val Ala Ser Ala Leu Met Val Glu Gln Lys Gly Ser Glu
1               5                   10                  15
GCA GCT GCT TCC AGC CCA GAC GTC TTG AGA GCG TGG GCG ACA CAG 90
Ala Ala Ala Ser Ser Pro Asp Val Leu Arg Ala Trp Ala Thr Gln
                20                  25                  30
TAT CAC ATG CCA TCC GAG TCG TCA GAC GCA GCT CGT CCT GCG CTA 135
Tyr His Met Pro Ser Glu Ser Ser Asp Ala Ala Arg Pro Ala Leu
                35                  40                  45
AAG CAC GCC TAC AAA CCT CCA GCA TCT GAC GCC AAG GGC ATC ACG 180
Lys His Ala Tyr Lys Pro Pro Ala Ser Asp Ala Lys Gly Ile Thr
                50                  55                  60
ATG GCG CTG ACC ATC ATT GGC ACC TGG ACC GCA GTG TTT TTA CAC 225
Met Ala Leu Thr Ile Ile Gly Thr Trp Thr Ala Val Phe Leu His
                65                  70                  75
GCA ATA TTT CAA ATC AGG CTA CCG ACA TCC ATG GAC CAG CTT CAC 270
Ala Ile Phe Gln Ile Arg Leu Pro Thr Ser Met Asp Gln Leu His
                80                  85                  90
TGG TTG CCT GTG TCC GAA GCC ACA GCC CAG CTT TTG GGC GGA AGC 315
Trp Leu Pro Val Ser Glu Ala Thr Ala Gln Leu Leu Gly Gly Ser
                95                  100                 105
AGC AGC CTA CTG CAC ATC GCT GCA GTC TTC ATT GTA CTT GAG TTC 360
Ser Ser Leu Leu His Ile Ala Ala Val Phe Ile Val Leu Glu Phe
                110                 115                 120
CTG TAC ACT GGT CTA TTC ATC ACC ACA CAT GAC GCA ATG CAT GGC 405
Leu Tyr Thr Gly Leu Phe Ile Thr Thr His Asp Ala Met His Gly
                125                 130                 135

ACC ATA GCT TTG AGG CAC AGG CAG CTC AAT GAT CTC CTT GGC AAC 450
Thr Ile Ala Leu Arg His Arg Gln Leu Asn Asp Leu Leu Gly Asn
                140                 145                 150
ATC TGC ATA TCA CTG TAC GCC TGG TTT GAC TAC AGC ATG CTG CAT 495
Ile Cys Ile Ser Leu Tyr Ala Trp Phe Asp Tyr Ser Met Leu His
                155                 160                 165
CGC AAG CAC TGG GAG CAC CAC AAC CAT ACT GGC GAA GTG GGG AAA 540
Arg Lys His Trp Glu His His Asn His Thr Gly Glu Val Gly Lys
                170                 175                 180
GAC CCT GAC TTC CAC AAG GGA AAT CCC GGC CTT GTC CCC TGG TTC 585
Asp Pro Asp Phe His Lys Gly Asn Pro Gly Leu Val Pro Trp Phe
                185                 190                 195
GCC AGC TTC ATG TCC AGC TAC ATG TCC CTG TGG CAG TTT GCC CGG 630
Ala Ser Phe Met Ser Ser Tyr Met Ser Leu Trp Gln Phe Ala Arg
                200                 205                 210
CTG GCA TGG TGG GCA GTG GTG ATG CAA ATG CTG GGG GCG CCC ATG 675
Leu Ala Trp Trp Ala Val Val Met Gln Met Leu Gly Ala Pro Met
                215                 220                 225
GCA AAT CTC CTA GTC TTC ATG GCT GCA GCC CCA ATC TTG TCA GCA 720
Ala Asn Leu Leu Val Phe Met Ala Ala Ala Pro Ile Leu Ser Ala
                230                 235                 240
TTC CGC CTC TTC TAC TTC GGC ACT TAC CTG CCA CAC AAG CCT GAG 765
Phe Arg Leu Phe Tyr Phe Gly Thr Tyr Leu Pro His Lys Pro Glu
                245                 250                 255
CCA GGC CCT GCA GCA GGC TCT CAG GTG ATG GCC TGG TTC AGG GCC 810
Pro Gly Pro Ala Ala Gly Ser Gln Val Met Ala Trp Phe Arg Ala
                260                 265                 270
AAG ACA AGT GAG GCA TCT GAT GTG ATG AGT TTC CTG ACA TGC TAC 855
Lys Thr Ser Glu Ala Ser Asp Val Met Ser Phe Leu Thr Cys Tyr

                275                 280                 285
CAC TTT GAC CTG CAC TGG GAG CAC CAC AGG TGG CCC TTT GCC CCC 900
His Phe Asp Leu His Trp Glu His His Arg Trp Pro Phe Ala Pro
                290                 295                 300
TGG TGG CAG CTG CCC CAC TGC CGC CGC CTG TCC GGG CGT GGC CTG 945
Trp Trp Gln Leu Pro His Cys Arg Arg Leu Ser Gly Arg Gly Leu
                305                 310                 315
GTG CCT GCC TTG GCA TGA 963
Val Pro Ala Leu Ala
                320

### INFORMATION FOR SEQ ID NO: 6

### SEQUENCE CHARACTERISTICS:

LENGTH: 942 base pairs

TYPE: nucleic acids

STRANDEDNESS: double

TOPOLOGY: linear

### MOLECULAR TYPE: CDNA

### SOURCE:

SPECIES: *Haematococcus pluvialis*

STRAIN: NIES-144

### SEQUENCE DESCRIPTION: SEQ ID NO: 6:

ATG GTC GAG CAG AAA GGC AGT GAG GCA GCT GCT TCC AGC CCA GAC 45
Met Val Glu Gln Lys Gly Ser Glu Ala Ala Ala Ser Ser Pro Asp
1               5                   10                  15
GTC TTG AGA GCG TGG GCG ACA CAG TAT CAC ATG CCA TCC GAG TCG 90
Val Leu Arg Ala Trp Ala Thr Gln Tyr His Met Pro Ser Glu Ser
                20                  25                  30

TCA GAC GCA GCT CGT CCT GCG CTA AAG CAC GCC TAC AAA CCT CCA 135
Ser Asp Ala Ala Arg Pro Ala Leu Lys His Ala Tyr Lys Pro Pro
                35                  40                  45
GCA TCT GAC GCC AAG GGC ATC ACG ATG GCG CTG ACC ATC ATT GGC 180
Ala Ser Asp Ala Lys Gly Ile Thr Met Ala Leu Thr Ile Ile Gly
                50                  55                  60
ACC TGG ACC GCA GTG TTT TTA CAC GCA ATA TTT CAA ATC AGG CTA 225
Thr Trp Thr Ala Val Phe Leu His Ala Ile Phe Gln Ile Arg Leu
                65                  70                  75
CCG ACA TCC ATG GAC CAG CTT CAC TGG TTG CCT GTG TCC GAA GCC 270
Pro Thr Ser Met Asp Gln Leu His Trp Leu Pro Val Ser Glu Ala
                80                  85                  90
ACA GCC CAG CTT TTG GGC GGA AGC AGC AGC CTA CTG CAC ATC GCT 315
Thr Ala Gln Leu Leu Gly Gly Ser Ser Ser Leu Leu His Ile Ala
                95                  100                 105
GCA GTC TTC ATT GTA CTT GAG TTC CTG TAC ACT GGT CTA TTC ATC 360
Ala Val Phe Ile Val Leu Glu Phe Leu Tyr Thr Gly Leu Phe Ile
                110                 115                 120
ACC ACA CAT GAC GCA ATG CAT GGC ACC ATA GCT TTG AGG CAC AGG 405
Thr Thr His Asp Ala Met His Gly Thr Ile Ala Leu Arg His Arg
                125                 130                 135
CAG CTC AAT GAT CTC CTT GGC AAC ATC TGC ATA TCA CTG TAC GCC 450
Gln Leu Asn Asp Leu Leu Gly Asn Ile Cys Ile Ser Leu Tyr Ala
                140                 145                 150
TGG TTT GAC TAC AGC ATG CTG CAT CGC AAG CAC TGG GAG CAC CAC 495
Trp Phe Asp Tyr Ser Met Leu His Arg Lys His Trp Glu His His
                155                 160                 165
AAC CAT ACT GGC GAA GTG GGG AAA GAC CCT GAC TTC CAC AAG GGA 540
Asn His Thr Gly Glu Val Gly Lys Asp Pro Asp Phe His Lys Gly

                170                 175                 180
AAT CCC GGC CTT GTC CCC TGG TTC GCC AGC TTC ATG TCC AGC TAC 585
Asn Pro Gly Leu Val Pro Trp Phe Ala Ser Phe Met Ser Ser Tyr
                185                 190                 195
ATG TCC CTG TGG CAG TTT GCC CGG CTG GCA TGG TGG GCA GTG GTG 630
Met Ser Leu Trp Gln Phe Ala Arg Leu Ala Trp Trp Ala Val Val
                200                 205                 210
ATG CAA ATG CTG GGG GCG CCC ATG GCA AAT CTC CTA GTC TTC ATG 675
Met Gln Met Leu Gly Ala Pro Met Ala Asn Leu Leu Val Phe Met
                215                 220                 225
GCT GCA GCC CCA ATC TTG TCA GCA TTC CGC CTC TTC TAC TTC GGC 720
Ala Ala Ala Pro Ile Leu Ser Ala Phe Arg Leu Phe Tyr Phe Gly
                230                 235                 240
ACT TAC CTG CCA CAC AAG CCT GAG CCA GGC CCT GCA GCA GGC TCT 765
Thr Tyr Leu Pro His Lys Pro Glu Pro Gly Pro Ala Ala Gly Ser
                245                 250                 255
CAG GTG ATG GCC TGG TTC AGG GCC AAG ACA AGT GAG GCA TCT GAT 810
Gln Val Met Ala Trp Phe Arg Ala Lys Thr Ser Glu Ala Ser Asp
                260                 265                 270
GTG ATG AGT TTC CTG ACA TGC TAC CAC TTT GAC CTG CAC TGG GAG 855
Val Met Ser Phe Leu Thr Cys Tyr His Phe Asp Leu His Trp Glu
                275                 280                 285
CAC CAC AGG TGG CCC TTT GCC CCC TGG TGG CAG CTG CCC CAC TGC 900
His His Arg Trp Pro Phe Ala Pro Trp Trp Gln Leu Pro His Cys
                290                 295                 300
CGC CGC CTG TCC GGG CGT GGC CTG GTG CCT GCC TTG GCA TGA 942
Arg Arg Leu Ser Gly Arg Gly Leu Val Pro Ala Leu Ala
305 310 313

### INFORMATION FOR SEQ ID NO: 7

### SEQUENCE CHARACTERISTICS:

LENGTH: 867 base pairs

TYPE: nucleic acids

STRANDEDNESS: double

TOPOLOGY: linear

### MOLECULAR TYPE: cDNA

### SOURCE:

SPECIES: *Haematococcus pluvialis*

STRAIN: NIES-144

### SEQUENCE DESCRIPTION: SEQ ID NO: 7:

ATG CCA TCC GAG TCG TCA GAC GCA GCT CGT CCT GCG CTA AAG CAC 45
Met Pro Ser Glu Ser Ser Asp Ala Ala Arg Pro Ala Leu Lys His
1               5                   10                  15
GCC TAC AAA CCT CCA GCA TCT GAC GCC AAG GGC ATC ACG ATG GCG 90
Ala Tyr Lys Pro Pro Ala Ser Asp Ala Lys Gly Ile Thr Met Ala
                20                  25                  30
CTG ACC ATC ATT GGC ACC TGG ACC GCA GTG TTT TTA CAC GCA ATA 135
Leu Thr Ile Ile Gly Thr Trp Thr Ala Val Phe Leu His Ala Ile
                35                  40                  45
TTT CAA ATC AGG CTA CCG ACA TCC ATG GAC CAG CTT CAC TGG TTG 180
Phe Gln Ile Arg Leu Pro Thr Ser Met Asp Gln Leu His Trp Leu
                50                  55                  60
CCT GTG TCC GAA GCC ACA GCC CAG CTT TTG GGC GGA AGC AGC AGC 225
Pro Val Ser Glu Ala Thr Ala Gln Leu Leu Gly Gly Ser Ser Ser
                65                  70                  75
CTA CTG CAC ATC GCT GCA GTC TTC ATT GTA CTT GAG TTC CTG TAC 270
Leu Leu His Ile Ala Ala Val Phe Ile Val Leu Glu Phe Leu Tyr

                80                  85                  90
ACT GGT CTA TTC ATC ACC ACA CAT GAC GCA ATG CAT GGC ACC ATA 315
Thr Gly Leu Phe Ile Thr Thr His Asp Ala Met His Gly Thr Ile
                95                  100                 105
GCT TTG AGG CAC AGG CAG CTC AAT GAT CTC CTT GGC AAC ATC TGC 360
Ala Leu Arg His Arg Gln Leu Asn Asp Leu Leu Gly Asn Ile Cys
                110                 115                 120
ATA TCA CTG TAC GCC TGG TTT GAC TAC AGC ATG CTG CAT CGC AAG 405
Ile Ser Leu Tyr Ala Trp Phe Asp Tyr Ser Met Leu His Arg Lys
                125                 130                 135
CAC TGG GAG CAC CAC AAC CAT ACT GGC GAA GTG GGG AAA GAC CCT 450
His Trp Glu His His Asn His Thr Gly Glu Val Gly Lys Asp Pro
                140                 145                 150
GAC TTC CAC AAG GGA AAT CCC GGC CTT GTC CCC TGG TTC GCC AGC 495
Asp Phe His Lys Gly Asn Pro Gly Leu Val Pro Trp Phe Ala Ser
                155                 160                 165
TTC ATG TCC AGC TAC ATG TCC CTG TGG CAG TTT GCC CGG CTG GCA 540
Phe Met Ser Ser Tyr Met Ser Leu Trp Gln Phe Ala Arg Leu Ala
                170                 175                 180
TGG TGG GCA GTG GTG ATG CAA ATG CTG GGG GCG CCC ATG GCA AAT 585
Trp Trp Ala Val Val Met Gln Met Leu Gly Ala Pro Met Ala Asn
                185                 190                 195
CTC CTA GTC TTC ATG GCT GCA GCC CCA ATC TTG TCA GCA TTC CGC 630
Leu Leu Val Phe Met Ala Ala Ala Pro Ile Leu Ser Ala Phe Arg
                200                 205                 210
CTC TTC TAC TTC GGC ACT TAC CTG CCA CAC AAG CCT GAG CCA GGC 675
Leu Phe Tyr Phe Gly Thr Tyr Leu Pro His Lys Pro Glu Pro Gly
                215                 220                 225
CCT GCA GCA GGC TCT CAG GTG ATG GCC TGG TTC AGG GCC AAG ACA 720

Pro Ala Ala Gly Ser Gln Val Met Ala Trp Phe Arg Ala Lys Thr
                230                 235                 240
AGT GAG GCA TCT GAT GTG ATG AGT TTC CTG ACA TGC TAC CAC TTT 765
Ser Glu Ala Ser Asp Val Met Ser Phe Leu Thr Cys Tyr His Phe
                245                 250                 255
GAC CTG CAC TGG GAG CAC CAC AGG TGG CCC TTT GCC CCC TGG TGG 810
Asp Leu His Trp Glu His His Arg Trp Pro Phe Ala Pro Trp Trp
                260                 265                 270
CAG CTG CCC CAC TGC CGC CGC CTG TCC GGG CGT GGC CTG GTG CCT 855
Gln Leu Pro His Cys Arg Arg Leu Ser Gly Arg Gly Leu Val Pro
                275                 280                 285
GCC TTG GCA TGA 867
Ala Leu Ala

## Claims

1. A DNA selected from the group consisting of (a) to (c):
(a) A DNA comprising the base sequence as shown in any one of SEQ ID 4, SEQ ID NO 5, SEQ ID NO 6,or SEQ ID NO 7,
(b) a DNA coding for the polypeptide which comprises the amino acid sequence as shown in any one of SEQ ID NO 1, SEQ ID NO 2, or SEQ ID NO 3,
(c) a DNA which hybridises with the DNA of (a) or (b) when reacted in a hybridisation solution comprising 6xDenhardt, 5xSSC, 0.2% SDS, 100µg/ml ssDNA at 55°C for 16 hours followed by washing with 2xSSC, 0.1% SDS at 60°C for 1 hour, wherein said DNA codes for a polypeptide having an enzyme activity to convert the methylene group at position 4 of a β-ionone ring in a compound containing β-ionone rings to a keto group.

2. The DNA according to claim 1 wherein said compound containing β-ionone rings is β-carotene.

3. The DNA of any one of claims 1 and 2 wherein a hydrogen atom at position 3 of said β-ionone ring may be replaced with a hydroxyl group.

4. The DNA of claim 3 wherein the compound containing said β-ionone ring where a hydrogen atom at position 3 is replaced with a hydroxyl group is zeaxanthin.

5. A DNA contained in the 1.7 kb region between the Sall and Xbal site of plasmid pHP51 which is contained in the bacterium having the accession No. FERM BP-4757, said DNA comprising a base sequence coding for a polypeptide having an enzyme activity to convert the methylene group at position 4 of the β-ionone ring in a compound containing β-ionone rings to a keto group.

6. A polypeptide having an enzyme activity to convert the methylene group at position 4 of a β-ionone ring in a compound containing β-ionone rings to a keto group which is encoded by a DNA sequence according to claims 1 to 4.

7. A recombinant vector comprising the DNA of claims 1 to 5.

8. A microorganism into which the DNA of claims 1 to 5 has been introduced.

9. A method for producing a ketocarotenoid, comprising culturing the microorganism of claim 8 which has inherently a group of carotenoid synthesis genes or into which such genes are introduced, in a medium and collecting a ketocarotenoid from the culture.

10. The method of claim 9 wherein said ketocarotenoid is at least one compound selected from the group consisting of echinenone and canthaxanthin.

11. The method of claim 9 wherein said ketocarotenoid is at least one compound selected from the group consisting of 4-ketozeaxanthin and astaxanthin.

12. The method of claim 9 wherein said microorganism of claim 8 is a bacterium or a yeast.

## Patentansprüche

1. DNS ausgewählt aus der Gruppe bestehend aus (a) bis (c):
a. DNS, die eine Basensequenz umfasst, die in irgend einer der SEQ ID Nr. 4, SEQ ID Nr. 5, SEQ ID Nr. 6 oder SEQ ID Nr. 7 gezeigt ist.
b. DNS, die ein Polypeptid kodiert, das die Aminosäuresequenz umfasst, die in irgend einer der SEQ ID Nr. 1, SEQ ID Nr. 2 oder SEQ ID Nr. 3 gezeigt ist.
c. DNS, die mit der DNS von (a) oder (b) hybridisiert, wenn sie bei 55 ° C 16 Stunden in einer Hybridisierungslösung reagiert, die 6 x Denhardt, 5 x SSC, 0,2 % SDS, 100 µg/ml ssDNS umfasst, gefolgt vom Waschen bei 60 °C 1 Stunde mit 2 x SSC, 0,1 % SDS, wobei die DNS ein Polypeptid mit der enzymatischen Aktivität zur Umwandlung einer Methylgruppe an Position 4 eines β-Ionon-Rings in eine Ketogruppe in einer β-Ionon-Ringe enthaltenden Verbindung kodiert.

2. DNS gemäß Patentanspruch 1, wobei die β-Ionon-Ringe enthaltende Verbindung β-Karotin ist.

3. DNS gemäß irgend einem der Patentansprüche 1 und 2, wobei ein Wasserstoffatom an Position 3 des β-Ionon-Rings durch eine Hydroxylgruppe ersetzt werden kann.

4. DNS gemäß Patentanspruch 3, wobei die den β-Ionon-Ring enthaltende Verbindung, in der ein Wasserstoffatom an Position 3 durch eine Hydroxylgruppe ersetzt wird, Zeaxanthin ist.

5. DNS, die in der 1,7 kb Region zwischen den Sal I- und Xba I-Schnittstellen des Plasmids pHP51 enthalten ist, welches in dem Bakterium mit der Zulassungsnummer FERM BP-4757 enthalten ist, und die DNS eine Basensequenz umfasst, welche für ein Polypeptid mit der enzymatischen Aktivität zur Umwandelung einer Methylgruppe an Position 4 eines β-Ionon-Rings in eine Ketogruppe in einer β-Ionon-Ringe enthaltenden Verbindung kodiert.

6. Polypeptid mit der enzymatischen Aktivität zur Umwandlung einer Methylgruppe an Position 4 eines β-Ionon-Rings in eine Ketogruppe in einer β-Ionon-Ringe enthaltenden Verbindung, welches durch die DNS-Sequenz gemäß Patentansprüchen 1 bis 4 kodiert ist.

7. Rekombinanter Vektor, der die DNS gemäß Patentansprüchen 1 bis 5 umfasst.

8. Mikroorganismus, in den die DNS gemäß Patentansprüchen 1 bis 5 eingeschleust worden ist.

9. Verfahren zur Herstellung eines Ketokarotinoids, umfassend das Kultivieren des Mikroorganismus gemäß Patentanspruch 8, welcher inhärent eine Gruppe von Karotinoidsynthesegenen besitzt oder in den solche Gene eingeschleust sind in einem Medium und das Absammeln des Ketokarotinoids aus der Kultur.

10. Methode gemäß Patentanspruch 9, wobei das Ketokarotinoid mindestens eine Verbindung ist, die aus der Gruppe ausgewählt wurde, die aus Echinenon und Canthaxanthin besteht.

11. Methode gemäß Patentanspruch 9, wobei das Ketokarotinoid mindestens eine Verbindung ist, die aus der Gruppe ausgewählt wurde, die aus 4-Ketozeaxanthin und Astaxanthin besteht.

12. Methode gemäß Patentanspruch 9, wobei der Mikroorganismus gemäß Patentanspruch 8 ein Bakterium oder eine Hefe ist.

## Revendications

1. ADN choisi dans le groupe constitué par (a) à (c):
(a) un ADN comprenant la séquence de bases indiquée par l'une quelconque des séquences SEQ ID N° 4, SEQ ID N° 5, SEQ ID N° 6 ou SEQ ID N° 7.
(b) un ADN codant pour le polypeptide comprenant la séquence d'aminoacides indiquée par l'une quelconque des séquences SEQ ID N° 1, SEQ ID N° 2 ou SEQ ID N° 3.
(c) un ADN qui s'hybride avec l'ADN de (a) ou (b) lorsqu'il est soumis à une réaction dans une solution d'hybridation comprenant de la solution de Denhardt 6×, du SSC 5×, 0,2 % de SDS, 100 µg/ml d'ADN simple brin à 55°C pendant 16 heures, suivie d'un lavage avec du SSC 2×, 0,1 % de SDS à 60°C pendant 1 heure, ledit ADN codant pour un polypeptide ayant une activité enzymatique transformant le groupe méthylène en position 4 d'un cycle β-ionone dans un composé contenant des cycles β-ionone en un groupe céto.

2. ADN selon la revendication 1, dans lequel ledit composé contenant des cycles β-ionone est le β-carotène.

3. ADN selon l'une quelconque des revendications 1 et 2, dans lequel un atome d'hydrogène en position 3 dudit cycle β-ionone peut être remplacé par un groupe hydroxyle.

4. ADN selon la revendication 3, dans lequel le composé contenant ledit cycle β-ionone dans lequel un atome d'hydrogène en position 3 est remplacé par un groupe hydroxyle est la zéaxanthine.

5. ADN contenu dans la région de 1,7 kb entre les sites SalI et XbaI du plasmide pHP51 contenu dans la bactérie ayant le n° d'accès FERM BP-4757, ledit ADN comprenant une séquence de bases codant pour un polypeptide ayant une activité enzymatique transformant le groupe méthylène en position 4 du cycle β-ionone dans un composé contenant des cycles β-ionone en un groupe céto.

6. Polypeptide ayant une activité enzymatique transformant le groupe méthylène en position 4 du cycle β-ionone dans un composé contenant des cycles β-ionone en un groupe céto, qui est codé par une séquence d'ADN selon les revendications 1 à 4.

7. Vecteur recombiné comprenant l'ADN des revendications 1 à 5.

8. Micro-organisme dans lequel a été introduit l'ADN des revendications 1 à 5.

9. Procédé de production d'un cétocaroténoïde, comprenant la culture dans un milieu du micro-organisme selon la revendication 8 ayant par nature un groupe de gènes de synthèse de caroténoïdes ou dans lequel ont été introduits de tels gènes, et la récupération d'un cétocaroténoïde à partir de la culture.

10. Procédé selon la revendication 9, dans lequel ledit cétocaroténoïde est au moins un composé choisi dans le groupe constitué par l'échinénone et la canthaxanthine.

11. Procédé selon la revendication 9, dans lequel ledit cétocaroténoïde est au moins un composé choisi dans le groupe constitué par la 4-cétozéaxanthine et l'astaxanthine.

12. Procédé selon la revendication 9, dans lequel ledit micro-organisme de la revendication 8 est une bactérie ou une levure.
